(19) Europäisches Patentamt
European Patent Office
Office européen des brevets

(11) EP 2 438 921 A1

(12) **EUROPEAN PATENT APPLICATION**

(43) Date of publication:
**11.04.2012 Bulletin 2012/15**

(21) Application number: **10187121.8**

(22) Date of filing: **11.10.2010**

(51) Int Cl.:
***A61K 31/704*** *(2006.01)* ***A61P 21/02*** *(2006.01)*
***A61P 29/00*** *(2006.01)* ***A61P 25/22*** *(2006.01)*
***A61P 25/08*** *(2006.01)* ***A61P 25/20*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Universität Innsbruck**
**6020 Innsbruck (AT)**
- **Universität Wien**
**1010 Wien (AT)**

(72) Inventors:
- **Stuppner, Hermann**
**6091, Götzens (AT)**
- **Hering, Steffen**
**1190, Wien (AT)**
- **Taferner, Barbara**
**1190, Wien (AT)**
- **Khom, Sophia**
**1170, Wien (AT)**
- **Cicek, Serhat Sezai**
**83395 Freilassing (AT)**

(74) Representative: **Vossius & Partner**
**Siebertstrasse 4**
**81675 München (DE)**

(54) **23-O-Acetylshengmanol-3-O-beta-D-xylopyranoside and its medical use**

(57) The present invention relates to 23-O-acetylshengmanol-3-O-β-D-xylopyranoside or a compound of formula (I) for use as a medicament, and pharmaceutical compositions comprising that compound, in particular for use as an anxiolytic agent, a sedative agent, a hypnotic agent, an anticonvulsant agent, an analgesic agent, an anesthetic agent, an antiepileptic agent, and/or a muscle relaxant.

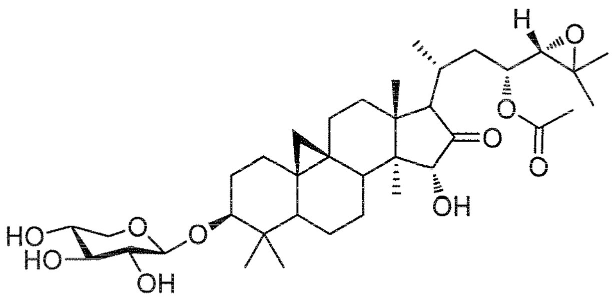

(I)

EP 2 438 921 A1

## Description

The present invention relates to 23-O-acetylshengmanol-3-O-β-D-xylopyranoside or a compound of formula (I) as described and defined herein for use as a medicament, and pharmaceutical compositions comprising that compound, in particular for use as an anxiolytic agent, a sedative agent, a hypnotic agent, an anticonvulsant agent, an analgesic agent, an anesthetic agent, an antiepileptic agent, and/or a muscle relaxant.

*Actaea racemosa* L. (syn.: *Cimicifuga* racemosa (L.) Nutt.), commonly known as black cohosh, is a perennial herb growing in temperate and cool temperate regions of the northern hemisphere (Britton, N et al. An illustrated flora of the Northern United States, Canada and the British Possessions - Vol. II. Charles Scribner's sons: New York, 1913). The centre of its distribution is in the south-eastern United States, where Native North American groups used the rhizome of black cohosh for the treatment of rheumatism and menstrual disorders (Britton, N et al. loc cit; Hardy, M. J. Am. Pharmaceut. Assoc. 2000, 40, 234-242). After being present in the U.S. Pharmacopoeia for more than 100 years, *A. racemosa* became popular in Europe in the middle of the last century, where it underwent several pharmacological and clinical investigations (Foster, S. Herbalgram 1999, 35-49). Though the plants active principle was extensively studied, its mechanism of action remained unclear (Palacio, C et al. Drugs Aging 2009, 26, 23-36). Initial animal experiments of black cohosh extracts suggested a hormone-like activity of the lipophilic components (e.g. triterpene glycosides), whereupon an estrogenic effect was postulated (Jarry, H et al. Planta Med. 1985, 46-49; Jarry, H et al. Planta Mend. 1985, 316-319; Düker, EM et al. Planta Med. 1991, 57, 420-424; Wuttke, W et al. Maturitas 2003, 44, 9-20), but latter studies disproved this theory (Zava, D et ai. Proc. Soc. Exp. Biol, Med. 1998, 217, 369-378; Liu, J et al. J. Agric. Food Chem. 2001, 49, 2472-2479; Zierau, O et al. J. Steroid Biochem. Mol. Biol. 2002, 80, 125-130; Amato, P et al. Menopause 2002, 9, 145-150; Bodinet, C et al. Breast Cancer Res. Treat. 2002, 76, 1-10; Onorato, J et al. Anal. Chem. 2001, 73, 4704-4710; Cheema et al., 2007; McBane, 2008; Uebelhack et al., 2006). There is, however, evidence for interaction of *A. racemosa* and its main constituents with the central nervous system (CNS) (Borelli, F et al. Pharmacol. Res. 2008, 58, 8-14; Palacio, C et al. Drugs Aging 2009, 26, 23-36). Such interactions have been suggested, including the central endogenous opioid system (Reame et al., 2008), the μ-opioid receptor (Rhyu et al., 2006), $5\text{-}HT_{1A}$, $5\text{-}HT_{1D}$ and $5\text{-}HT_7$ receptors (Burdette et al., 2003) or $D_2$ receptors (Jarry et al., 2003). Furthermore, there is accumulating evidence that black cohosh preparations are more effective than placebo in alleviating moderate vasomotor symptoms associated in the menopause (Cheema et al., 2007; McBane, 2008, Uebelhack et a!., 2006). In addition, an improvement in sleep and mood disturbances was observed (Cheema et al., 2008).

Previous studies resulted in the quantitative determination of the compounds **1**, **3** and **5** as defined below in black cohosh extracts (Ganzera et al., 2000, Avula et al., 2009) and the identification of cycloartane glycosides in several *Actaea*/*Cimicifuga* species (He et al., 2006, Avula et al., 2007). Specific terpenoids isolated from *Cimicifuga foetida* L. are described, e.g., in JP-A-09/031094.

γ-Aminobutyric acid (GABA) type A ($GABA_A$) receptors mediate the inhibitory neurotransmission in the mammalian CNS (Nadler et al, 1977; Sieghart 1995; Hevers and Lüddens, 1998, Michels and Moss, 2007). They represent ligand gated chloride channels that are assembled from different subunits forming a pentameric structure. Nineteen subunits of mammalian $GABA_A$ receptors have been cloned so far: $\alpha_{1\text{-}6}$, $\beta_{1\text{-}3}$, $\gamma_{1\text{-}3}$, δ, ε, π, $\rho_{1\text{-}3}$ and θ (Barnard et al., 1998; Simon et al., 2004). Consequently, a large variety of different receptor subtypes can be formed (Olsen and Sieghart, 2008). $GABA_A$ receptors represent the molecular target of multiple clinically important drugs such as BZDs, barbiturates and general anaesthetics (see Sieghart 2006 for review, Möhler, 2006; Sieghart and Olsen, 2008), but also natural compounds of plant origin including flavonoids, e.g. methyl-apigenin (Marder et al., 2003), wogonin (from *Scutellaria baicalensis,* Hui et al., 2002; Huen et al., 2003) or 6,3'-Dinitroflavone (Furtmueller et al., 2008), monoterpenes, e.g. borneol (Granger et al., 2005) and thymol (Garcia et al., 2005), polyacteylenes e.g. MS-1, MS-2 and MS-4 (from *Cussonia zimmermannii,* Baur et al., 2005), and valerenic acid (from *Valeriana officinalis,* Khom et al., 2007; Benke et al. 2009).

In the present invention, the interaction of black cohosh extracts with $GABA_A$ receptors was analysed. In order to analyse the effect of these extracts on GABA-induced chloride currents ($I_{GABA}$) through GABA preceptors, receptors composed of 2 $\alpha_1$-, 2 $\beta_2$- and 1 $\gamma_{2S}$-subunit were expressed in *Xenopus laevis* oocytes. Modulation of $I_{GABA}$ was studied by means of the two-microelectrode voltage-clamp technique.

In accordance with Example 1 and as also shown in Figure 1, the following 11 cycloartane glycosides (compounds **1** to **11**) were isolated and identified:

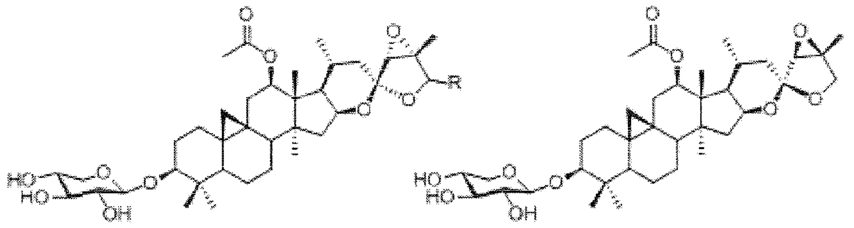

1 R=OH
2 R=H

3

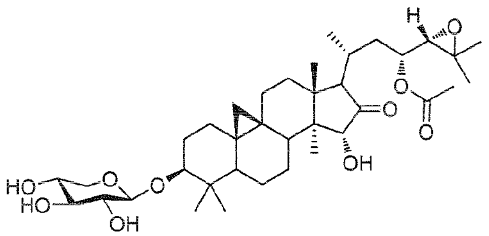

4

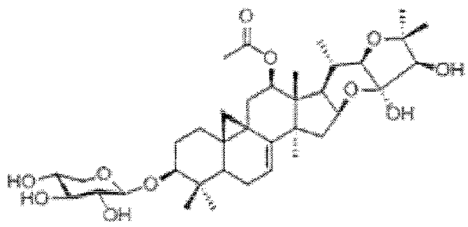

5

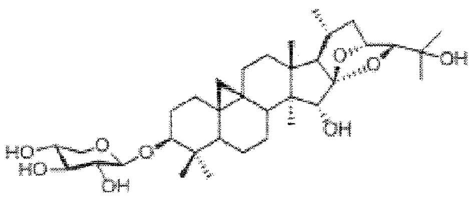

6

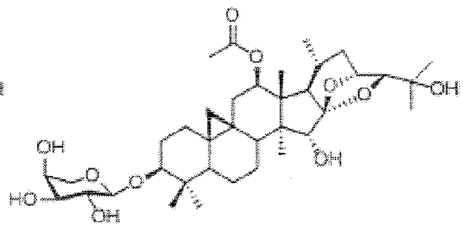

7

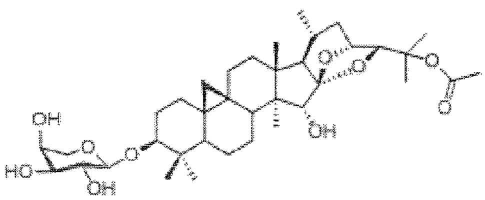

8

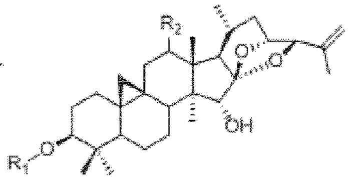

9 $R_1$=α-L-ara, $R_2$=H
10 $R_1$=β-D-xyl, $R_2$=H
11 $R_1$=β-D-xyl, $R_2$=OAc

Compounds **1**, **4**, **6**, and **8** significantly enhance GABA-induced chloride currents ($I_{GABA}$) by more than 150% at 100 μM, when co-applied with GABA ($EC_{3-10}$), as determined in Example 1 and shown in Figure 4.

It was surprisingly found that compound **4** (i.e., 23-O-acetylshengmanol-3-O-β-D-xylopyranoside) induces an about 10-times stronger $I_{GABA}$ enhancement than the other isolated compounds, including compound **10.** Accordingly, compound **4** is extraordinarily effective in enhancing GABA-induced chloride currents and, thus, is particularly useful as a positive allosteric $GABA_A$ receptor modulator.

The established allosteric modulation of $GABA_A$ receptors by the identified compounds and, in particular, by compound **4** causes, e.g., anxiolytic, sedative and hypnotic effects that potentially contribute to beneficial effects described for black cohosh extracts in the treatment of postmenopausal symptoms.

Compound **4**, i.e. 23-O-acetylshengmanol-3-O-β-D-xylopyranoside, is thus useful as an anxiolytic agent, a

sedative agent, a hypnotic agent, an anticonvulsant agent, an analgesic agent, an anesthetic agent, an antiepileptic agent, and/or a muscle relaxant, and also in the treatment or prevention of climacteric/postmenopausal symptoms.

Accordingly, the present invention relates to 23-O-acetylshengmanol-3-O-β-D-xylopyranoside (CAS number: 62498-88-8) or a compound of the following formula (I)

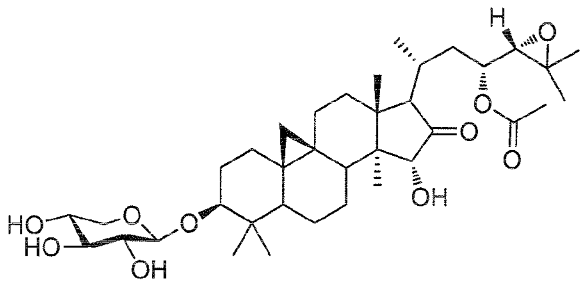

(I)

or an enantiomer or diastereomer thereof, or a pharmaceutically acceptable salt, solvate or prodrug thereof, for use as a medicament.

The invention further relates to a pharmaceutical composition comprising 23-O-acetylshengmanol-3-O-R-D-xylopyranoside or the compound of formula (I), as defined herein, or an enantiomer or diastereomer thereof, or a pharmaceutically acceptable salt, solvate or prodrug thereof, and a pharmaceutical acceptable excipient.

In particular, the present invention relates to 23-O-acetylshengmanol-3-O-β-D-xylopyranoside (CAS number: 62498-88-8) or the compound of formula (I), as defined herein, or an enantiomer or diastereomer thereof, or a pharmaceutical acceptable salt, solvate or prodrug thereof, or a pharmaceutical composition comprising any of the aforementioned entities and a pharmaceutically acceptable excipient, for use as an anxiolytic agent, a sedative agent, a hypnotic agent, an anticonvulsant agent, an analgesic agent, an anesthetic agent, an antiepileptic agent, and/or a muscle relaxant (and, in particular, for use as an anxiolytic agent, a sedative agent, or a hypnotic agent). As shown in Example 1, 23-O-acetylshengmanol-3-O-β-D-xylopyranoside, i.e. the compound of formula (I), is particularly effective in enhancing GABA-induced chloride currents and, accordingly, is particularly effective as a positive allosteric modulator of $GABA_A$ receptors which makes it useful as an anxiolytic agent, a sedative agent, a hypnotic agent, an anticonvulsant agent, an analgesic agent, an anesthetic agent, an antiepileptic agent, and/or a muscle relaxant. The particular effectiveness of 23-O-acetylshengmanol-3-O-β-D-xylopyranoside, i.e. the compound of formula (I), as, e.g. a sedative agent and/or as an anxiolytic agent has further been demonstrated *in vivo,* as described in Example 2.

23-O-Acetylshengmanol-3-O-β-D-xylopyranoside or the compound of formula (I) is thus useful in the medical intervention of, e.g., insomnia, anxiety, pain, mood disorders, panic disorders, epilepsy, schizophrenia, disorders/symptoms connected to or associated with alcohol and/or substance withdrawal or abuse, and climacteric or postmenopausal symptoms, as is evident from Examples 1 and 2. Accordingly, the invention relates to 23-O-acetylshengmanol-3-O-β-D-xylopyranoside (CAS number: 62498-88-8) or the compound of formula (I), as defined herein, or an enantiomer or diastereomer thereof, or a pharmaceutically acceptable salt, solvate or prodrug thereof, or a pharmaceutical composition comprising any of the aforementioned entities and a pharmaceutically acceptable excipient, for use in the treatment or prevention of: insomnia; anxiety; pain disoders; mood disorders; panic disorders; epilepsy; schizophrenia; disorders/symptoms connected to or associated with alcohol and/or substance withdrawal or abuse; or climacteric or postmenopausal symptoms.

The invention furthermore relates to 23-O-acetylshengmanol-3-O-β-D-xylopyranoside (CAS number: 62498-88-8) or the compound of formula (I), as defined herein, or an enantiomer or diastereomer thereof, or a pharmaceutically acceptable salt, solvate or prodrug thereof, or a pharmaceutical composition comprising any of the aforementioned entities and a pharmaceutically acceptable excipient, for use in the treatment or prevention of: anxiety; anxiety disorders, such as, e.g., generalized or substance-induced anxiety disorder, panic disorder with or without agoraphobia, agoraphobia without history of panic disorder, phobias (including, e.g., social phobias), or obsessive-compulsive disorder; Down Syndrome; sleep disorders, cognitive disorders and seizure disorders, such as, e.g., epilepsy or pain; depression or bipolar disorders, such as, e.g., single-episode or recurrent major depressive disorder, dysthymic disorder, bipolar I manic disorder, or bipolar II manic disorder; neuroses; schizophrenia; attention deficit hyperactivity disorder; disorders of circadian rhythm, e.g., in subjects suffering from the effects of jet lag or shift work; overdose with benzodiazepine drugs; disorders/conditions including the impairment of recent memory and/or remote memory and/or alertness; Huntington's Chorea; muscular spasms and rigidity; withdrawal symptoms; Alzheimer's disease; Parkinson's disease; or

stress disorders, such as, e.g., post-traumatic and/or acute stress disorder.

In accordance with the invention, 23-O-acetylshengmanol-3-O-β-D-xylopyranoside or the compound of formula (I), as defined herein, or an enantiomer or diastereomer thereof, or a pharmaceutically acceptable salt, solvate or prodrug thereof, or a pharmaceutical composition comprising any of the aforementioned entities and a pharmaceutically acceptable excipient, can be used as general anaesthetics.

The present invention also reflates to a method of treating or preventing a disease, disorder or condition, the method comprising the administration of 23-O-acetylshengmanol-3-O-β-D-xylopyranoside (CAS number: 62498-88-8) or the compound of formula (I), as defined herein, or an enantiomer or diastereomer thereof, or a pharmaceutically acceptable salt, solvate or prodrug thereof, or a pharmaceutical composition comprising any of the aforementioned entities and a pharmaceutically acceptable excipient, to a subject (preferably, a human) in need of such a treatment or prevention. The disease, disorder or condition to be treated or prevented in accordance with the invention may be selected from, for example: insomnia; anxiety; pain; mood disorders; panic disorders; epilepsy; schizophrenia; disorders/symptoms connected to or associated with alcohol and/or substance withdrawal or abuse; or climacteric or postmenopausal symptoms. The disease, disorder or condition to be treated or prevented may also be selected from, for example: anxiety; anxiety disorders, such as, e.g., generalized or substance-induced anxiety disorder, panic disorder with or without agoraphobia, agoraphobia without history of panic disorder, phobias (including, e.g., social phobias), or obsessive-compulsive disorder; Down Syndrome; sleep disorders, cognitive disorders and seizure disorders, such as, e.g., epilepsy or pain; depression or bipolar disorders, such as, e.g., single-episode or recurrent major depressive disorder, dysthymic disorder, bipolar I manic disorder, or bipolar II manic disorder; neuroses; schizophrenia; attention deficit hyperactivity disorder; disorders of circadian rhythm, e.g., in subjects suffering from the effects of jet lag or shift work; overdose with benzodiazepine drugs; disorders/conditions including the impairment of recent memory and/or remote memory and/or alertness; Huntington's Chorea; muscular spasms and rigidity; withdrawal symptoms; Alzheimer's disease; Parkinson's disease; or stress disorders, such as, e.g., post-traumatic and/or acute stress disorder.

The invention further relates to the use of 23-O-acetylshengmanol-3-O-β-D-xylopyranoside or the compound of formula (I), as defined herein, or an enantiomer or diastereomer thereof, or a pharmaceutical acceptable salt, solvate or prodrug thereof, as a positive allosteric modulator of $GABA_A$ receptors.

Compound **4** (i.e., 23-O-acetylshengmanol-3-O-β-D-xylopyranoside) or the compound of formula (I) can be isolated from *Actaea racemosa,* as described in Example 1, or can be obtained commercially (e.g., from ChromaDex, Inc., Irvine, CA, USA).

The present invention furthermore relates to a compound of the following formula (II)

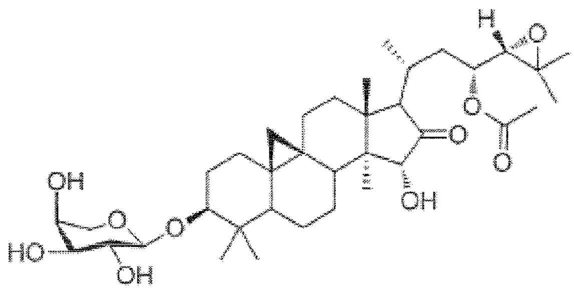

(II)

or an enantiomer or diastereomer thereof, or a pharmaceutical acceptable salt, solvate or prodrug thereof, for use as a medicament.

The invention also relates to a pharmaceutical composition comprising the compound of formula (II), as defined herein, or an enantiomer or diastereomer thereof, or a pharmaceutically acceptable salt, solvate or prodrugs thereof, and a pharmaceutically acceptable excipient.

In particular, the present invention relates to the compound of formula (II), as defined herein, or an enantiomer or diastereomer thereof, or a pharmaceutically acceptable salt, solvate or prodrug thereof, or a pharmaceutical composition comprising any of the aforementioned entities and a pharmaceutically acceptable excipient, for use as an anxiolytic agent, a sedative agent, a hypnotic agent, an anticonvulsant agent, an analgesic agent, an anesthetic agent, an antiepileptic agent, and/or a muscle relaxant.

The compound of formula (II) is useful in the medical intervention of, e.g., insomnia, anxiety, pain, mood disorders, panic disorders, epilepsy, schizophrenia, disorders/symptoms connected to or associated with alcohol and/or substance withdrawal or abuse, and climacteric or postmenopausal symptoms. Accordingly, the invention relates to the

compound of formula (II), as defined herein, or an enantiomer or diastereomer thereof, or a pharmaceutically acceptable salt, solvate or prodrug thereof, or a pharmaceutical composition comprising any of the aforementioned entities and a pharmaceutical acceptable excipient, for use in the treatment or prevention of: insomnia; anxiety; pain disoders; mood disorders; panic disorders; epilepsy; schizophrenia; disorders/symptoms connected to or associated with alcohol and/or substance withdrawal or abuse; or climacteric or postmenopausal symptoms.

The invention furthermore relates to the compound of formula (II), as defined herein, or an enantiomer or diastereomer thereof, or a pharmaceutical acceptable salt, solvate or prodrug thereof, or a pharmaceutical composition comprising any of the aforementioned entities and a pharmaceutically acceptable excipient, for use in the treatment or prevention of: anxiety; anxiety disorders, such as, e.g., generalized or substance-induced anxiety disorder, panic disorder with or without agoraphobia, agoraphobia without history of panic disorder, phobias (including, e.g., social phobias), or obsessive-compulsive disorder; Down Syndrome; sleep disorders, cognitive disorders and seizure disorders, such as, e.g., epilepsy or pain; depression or bipolar disorders, such as, e.g., single-episode or recurrent major depressive disorder, dysthymic disorder, bipolar I manic disorder, or bipolar II manic disorder; neuroses; schizophrenia; attention deficit hyperactivity disorder; disorders of circadian rhythm, e.g., in subjects suffering from the effects of jet lag or shift work; overdose with benzodiazepine drugs; disorders/conditions including the impairment of recent memory and/or remote memory and/or alertness; Huntington's Chorea; muscular spasms and rigidity; withdrawal symptoms; Alzheimer's disease; Parkinson's disease; or stress disorders, such as, e.g., post-traumatic and/or acute stress disorder.

The present invention also relates to a method of treating or preventing a disease, disorder or condition, the method comprising the administration of the compound of formula (II), as defined herein, or an enantiomer or diastereomer thereof, or a pharmaceutically acceptable salt, solvate or prodrug thereof, or a pharmaceutical composition comprising any of the aforementioned entities and a pharmaceutically acceptable excipient, to a subject (preferably, a human) in need of such a treatment or prevention. The disease, disorder or condition to be treated or prevented in accordance with the invention may be selected from, for example: insomnia; anxiety; pain; mood disorders; panic disorders; epilepsy; schizophrenia; disorders/symptoms connected to or associated with alcohol and/or substance withdrawal or abuse; or climacteric or postmenopausal symptoms. The disease, disorder or condition to be treated or prevented may also be selected from, for example: anxiety; anxiety disorders, such as, e.g., generalized or substance-induced anxiety disorder, panic disorder with or without agoraphobia, agoraphobia without history of panic disorder, phobias (including, e.g., social phobias), or obsessive-compulsive disorder; Down Syndrome; sleep disorders, cognitive disorders and seizure disorders, such as, e.g., epilepsy or pain; depression or bipolar disorders, such as, e.g., single-episode or recurrent major depressive disorder, dysthymic disorder, bipolar I manic disorder, or bipolar II manic disorder; neuroses; schizophrenia; attention deficit hyperactivity disorder; disorders of circadian rhythm, e.g., in subjects suffering from the effects of jet lag or shift work; overdose with benzodiazepine drugs; disorders/conditions including the impairment of recent memory and/or remote memory and/or alertness; Huntington's Chorea; muscular spasms and rigidity; withdrawal symptoms; Alzheimer's disease; Parkinson's disease; or stress disorders, such as, e.g., post-traumatic and/or acute stress disorder.

The invention further relates to the use of the compound of formula (II), as defined herein, or an enantiomer or diastereomer thereof, or a pharmaceutically acceptable salt, solvate or prodrug thereof, as a positive allosteric modulator of $GABA_A$ receptors.

The compound of formula (II) can be isolated from *Actaea racemosa* in analogy to the procedure described in Example 1.

The scope of the invention embraces all pharmaceutically acceptable salt forms of 23-O-acetylshengmanol-3-O-β-D-xylopyranoside or the compound of formula (I) or the compound of formula (II) which may be formed, e.g., by protonation of an atom carrying an electron lone pair which is susceptible to protonation, such as an amino group, with an inorganic or organic acid, or as a salt of a carboxylic acid group with a physiologically acceptable cation as they are well-known in the art. Exemplary base addition salts comprise, for example, alkali metal salts such as sodium or potassium salts; alkaline earth metal salts such as calcium or magnesium salts; ammonium salts; aliphatic amine salts such as trimethylamine, triethylamine, dicyclohexylamine, ethanolamine, diethanolamine, triethanolamine, procaine salts, meglumine salts, diethanol amine salts or ethylenediamine salts; aralkyl amine salts such as N,N-dibenzylethylenediamine salts, benetamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts, and basic amino acid salts such as arginine salts or lysine salts. Exemplary acid addition salts comprise, for example, mineral acid salts such as hydrochloride, hydrobromide, hydroiodide, sulfate salts, nitrate salts, phosphate salts (such as, e.g., phosphate, hydrogenphosphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate salts; sulfonate salts such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate salts; and acidic amino acid salts such as aspartate or glutamate salts.

Moreover, the scope of the invention embraces solid forms of 23-O-acetylshengmanol-3-O-β-D-xylopyranoside or the compound of formula (I) or the compound of formula (II) in any solvated form, including e.g. solvates with water, for example hydrates, or with organic solvents such as, e.g., methanol, ethanol or acetonitrile, i.e. as a methanolate, ethanolate or acetonitrilate, respectively; or in the form of any polymorph.

Furthermore, all stereoisomers of 23-O-acetylshengmanol-3-O-β-D-xylopyranoside or the compound of formula (I) or the compound of formula (II) are contemplated as part of the present invention, either in admixture or in pure or substantially pure form. The scope of the compounds according to the invention embraces all the possible stereoisomers and their mixtures. It very particularly embraces the racemic forms and the isolated optical isomers. The racemic forms can be resolved by physical methods, such as, e.g., fractional crystallization, separation or crystallization of diastereomeric derivatives or separation by chiral column chromatography. The individual optical isomers can be obtained from the racemates using conventional methods, such as, e.g., salt formation with an optically active acid followed by crystallization.

Pharmaceutically acceptable prodrugs of 23-O-acetylshengmanol-3-O-β-D-xylopyranoside or the compound of formula (I) or the compound of formula (II) are derivatives which have chemically or metabolically cleavable groups and become, by solvolysis or under physiological conditions, the compounds of the present invention which are pharmaceutically active in vivo. Prodrugs of compounds of the present invention may be formed in a conventional manner with a functional group of the compounds, in particular a hydroxy group. The prodrug derivative form often offers advantages of solubility, tissue compatibility or delayed release in a mammalian organism (see, Bundgaard, H., Design of Prodrugs, pp. 7-9, 21-24, Elsevier, Amsterdam 1985). Prodrugs according to the present invention include, for example, acyloxy derivatives prepared by reacting a hydroxyl group of 23-O-acetylshengmanol-3-O-β-D-xylopyranoside or the compound of formula (I) or the compound of formula (II) with a suitable acylhalide or a suitable acid anhydride. An especially preferred acyloxy derivative as a prodrug is $-OC(=O)-CH_3$, $-OC(=O)-C_2H_5$, -OC(=O)-(tert-Bu), $-OC(=O)-C_{15}H_{31}$, -OC(=O)-(m-COONa-Ph), $-OC(=O)-CH_2CH_2COONa$, $-O(C=O)-CH(NH_2)CH_3$ or $-OC(=O)-CH_2-N(CH_3)_2$. Accordingly, preferred prodrugs of 23-O-acetylshengmanol-3-O-β-D-xylopyranoside or the compound of formula (I) or the compound of formula (II) are such compounds, wherein one or more hydroxy groups are replaced by $-OC(=O)-CH_3$, $-OC(=O)-C_2H_5$, -OC(=O)-(tert-Bu), $-OC(=O)-C_{15}H_{31}$, -OC(=O)-(m-COONa-Ph), $-OC(=O)-CH_2CH_2COONa$, $-O(C=O)-CH(NH_2)CH_3$ or $-OC(=O)-CH_2-N(CH_3)_2$.

The compounds described herein may be administered as compounds per se in their use as pharmacophores or pharmaceutical compositions or may be formulated as medicaments. Within the scope of the present invention are pharmaceutical compositions comprising as an active ingredient 23-O-acetylshengmanol-3-O-β-D-xylopyranoside or the compound of formula (I) or the compound of formula (II) as defined above. The pharmaceutical compositions may optionally comprise one or more pharmaceutically acceptable excipients, such as carriers, diluents, fillers, disintegrants, lubricating agents, binders, colorants, pigments, stabilizers, preservatives, or antioxidants.

The pharmaceutical compositions can be formulated by techniques known to the person skilled in the art, such as the techniques published in Remington's Pharmaceutical Sciences, 20th Edition. The pharmaceutical compositions can be formulated as dosage forms for oral, parenteral, such as intramuscular, intravenous, subcutaneous, intradermal, intraarterial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds according to the invention, in particular 23-O-acetylshengmanol-3-O-β-D-xylopyranoside or the compound of formula (I) or the compound of formula (II), or the above described pharmaceutical compositions comprising 23-O-acetylshengmanol-3-O-β-D-xylopyranoside or the compound of formula (I) or the compound of formula (II) may be administered to a subject by any convenient route of administration, whether systemically/peripherally or at the site of desired action, including but not limited to one or more of: oral (e.g. as a tablet, capsule, or as an ingestible solution), topical (e.g., transdermal, intranasal, ocular, buccal, and sublingual), parenteral (e. g., using injection techniques or infusion techniques, and including, for example, by injection, e.g. subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g. through mouth or nose), gastrointestinal, intrauterine, intraocular, subcutaneous, ophthalmic (including intravitreal or intracameral), rectal, and vaginal.

If said compounds or pharmaceutical compositions are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

Alternatively, said compounds or pharmaceutical compositions can be administered in the form of a suppository or pessary, or it may be applied topically in the form of a gel, hydrogel, lotion, solutions, cream, ointment or dusting powder. The compounds of the present invention may also be dermally or transdermally administered, for example, by the use of a skin patch.

Said compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of 23-O-acetylshengmanol-3-O-β-D-xylopyranoside or the compound of formula (I) or the compound of formula (II) for administration to a human may be 0.02 to 2000 mg/kg body weight, preferably 0.2 mg to 500 mg/kg body weight, of the active ingredient per unit dose to achieve anxiolytic effects. Sedative effects by 23-O-acetylshengmanol-3-O-β-D-xylopyranoside or the compound of formula (I) or the compound of formula (II) will occur at higher doses. In particular, if 23-O-acetylshengmanol-3-O-β-D-xylopyranoside or the compound of formula (I) or the compound of formula (II) is used as a sedative agent, the minimal dose will be about 10 times higher than the dose of the respective compound when used as an anxiolytic agent. Sedative doses may be 2 mg to 2000 mg/kg bodyweight, preferably 6 mg to 600 mg/kg bodyweight. The unit dose may be administered, for example, 1 to 4 times per day. The dose will depend on the route of administration. It will be appreciated that it may be necessary to make routine variations to the dosage depending on the age and weight of the patient/subject as well as the severity of the condition to be treated. The precise dose and route of administration will ultimately be at the discretion of the attendant physician or veterinarian. For example, 23-O-acetylshengmanol-3-O-β-D-xylopyranoside or the compound of formula (I) or the compound of formula (II) may be administered prior to sleep, in particular in the treatment or prevention of sleep disorders or insomnia. 23-O-acetylshengmanol-3-O-β-D-xylopyranoside or the compound of formula (I) or the compound of formula (II) may also be administered, e.g., prior to surgery.

23-O-acetylshengmanol-3-O-β-D-xylopyranoside or the compound of formula (I) or the compound of formula (II) or a pharmaceutically acceptable salt or solvate thereof may, for example, be administered to a human patient in an amount of about 0.1 mg/kg body weight to about 10000 mg/kg body weight, preferably of about 1 mg/kg body weight to about 2500 mg/kg bodyweight per day. A human patient (of approximately 70 kg body weight) may, in particular, be treated with about 500 mg to about 1500 mg and, more specifically, with about 1000 mg per day of 23-O-acetylshengmanol-3-O-β-D-xylopyranoside or the compound of formula (I) or the compound of formula (II) or a pharmaceutically acceptable salt or solvent thereof.

Particularly preferred is the formulation of a medicament for oral application. In an embodiment where 23-O-acetylshengmanol-3-O-β-D-xylopyranoside or the compound of formula (I) or the compound of formula (II) is used for oral application, for example, about 0.2 to about 20000 mg, about 0.2 to about 6000 mg or about 0.2 to about 3000 mg of 23-O-23-O-acetylshengmanol-3-O-β-D-xylopyranoside or the compound of formula (I) or the compound of formula (II) or a pharmaceutically acceptable salt or solvate thereof may be used.

The compounds of the present invention, in particular 23-O-acetylshengmanol-3-O-β-D-xylopyranoside or the compound of formula (I) or the compound of formula (II), can be used in combination with other therapeutic agents. When a compound of the invention is used in combination with a second therapeutic agent active against the same disease, the dose of each compound may differ from that when the compound is used alone. The combination of a compound of this invention with another drug may comprise the administration of the drug with the compound of the invention. Such an administration may comprise simultaneous/concomitant administration. However, also sequential/ separate administration is envisaged.

The combinations referred to above may conveniently be presented for use in the form of a pharmaceutical formulation. The individual components of such combinations may be administered either sequentially or simultaneously/ concomitantly in separate or combined pharmaceutical formulations by any convenient route. When administration is sequential, either the present compound or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in any convenient formulation, conveniently in such manner as are known for such compounds in the art.

The term "treatment of a disorder or disease" as used herein, such as "treatment of climacteric or postmenopausal symptoms", is well known in the art. "Treatment of a disorder or disease" implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

"Treatment of a disorder or disease" may, for example, lead to a halt in the progression of the disorder or disease (e.g., no deterioration of symptoms) or a delay in the progression of the disorder or disease (in case the halt in progression is of a transient nature only). "Treatment of a disorder or disease" may also lead to a partial response (e.g., amelioration of symptoms) or complete response (e.g., disappearance of symptoms) of the subject/patient suffering from the disorder or disease. "Amelioration" of a disorder or disease may, for example, lead to a halt in the progression of the disorder or disease or a delay in the progression of the disorder or disease. Such a partial or complete response may be followed by a relapse. It is to be understood that a subject/patient may experience a broad range of responses to a treatment (e.g., the exemplary responses as described herein above).

Treatment of a disorder or disease may, inter alia, comprise curative treatment (preferably leading to a complete response and eventually to healing of the disorder or disease) and palliative treatment (including symptomatic relief).

Also the term "prevention of a disorder or disease" as used herein, such as "prevention of climacteric or postmenopausal symptoms", is well known in the art. For example, a patient/subject suspected of being prone to suffer from a disorder or disease as defined herein may, in particular, benefit from a prevention of the disorder or disease. The subject/patient may have a susceptibility or predisposition for a disorder or disease, including but not limited to hereditary predisposition. Such a predisposition can be determined by standard assays, using, for example, genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of compounds of the present invention before any clinical and/or pathological symptoms are diagnosed or determined or can be diagnosed or determined by the attending physician.

The subject or patient, such as the subject in need of treatment or prevention, may be a eukaryote, an animal, a vertebrate animal, a mammal, a rodent (e.g. a guinea pig, a hamster, a rat, a mouse), a murine (e.g. a mouse), a canine (e.g. a dog), a feline (e.g. a cat), an equine (e.g. a horse), a primate, a simian (e.g. a monkey or ape), a monkey (e.g. a marmoset, a baboon), an ape (e. g. gorilla, chimpanzee, orangutang, gibbon), or a human. The meaning of the terms "eukaryote", "animal", "mammal", etc. is well known in the art and can, for example, be deduced from Wehner und Gearing (1995; Thieme Venag). In the context of this invention, it is particularly envisaged that animals are to be treated which are economically, agronomically or scientifically important. Scientifically important organisms include, but are not limited to, mice, rats, and rabbits. Lower organisms such as, e.g., fruit flies like *Drosophila melagonaster* and nematodes like *Caenorhabditis elegant* may also be used in scientific approaches. Non-limiting examples of agronomically important animals are sheep, cattle and pig, while, for example, cats and dogs may be considered as economically important animals. Preferably, the subject/patient is a mammal; more preferably, the subject/patient is a human.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention also illustrated by the following illustrative figures. The appended figures show:

**Figure 1.** Extraction and isolation scheme of compounds **1-11** from *A. racemosa*.

**Figure 2.** Modulation of $I_{GABA}$ through $GABA_A$ receptors composed of $\alpha_1$, $\beta_2$ and $\gamma_{2S}$ subunits by extracts A0-A4. Bars illustrate $I_{GABA}$ modulation upon co-application of GABA ($EC_{3-10}$) and 100 μg/ml of the indicated extract. Each bar represents the mean ± S.E. from at least 3 oocytes and ≥ 2 oocyte batches. (*) indicates significantly different from zero ($p < 0.05$, *t*-test by ANOVA).

**Figure 3.** Modulation of $I_{GABA}$ through $GABA_A$ receptors composed of $\alpha_1$, $\beta_2$ and $\gamma_{2S}$ subunits by fractions A1A-A10. Bars illustrate the effect on $I_{GABA}$ upon co-application of GABA ($EC_{3-10}$) and 100 μg/ml of the indicated fraction. Each bar represents the mean ± S.E. from at least 3 oocytes and ≥ 2 oocyte batches. (*) indicates significantly different from zero ($p < 0.05$, *t*-test by ANOVA).

**Figure 4.** Modulation of $I_{GABA}$ in % by the indicated compounds isolated from black cohosh. Bars illustrate the enhancement of $I_{GABA}$ upon co-application of GABA ($EC_{3-10}$) and 100 μM of the indicated compounds. Each bar represents the mean ± S.E.M. from at least 3 oocytes and ≥ 2 oocyte batches. (*) indicates significantly different from zero ($p < 0.05$, *t*-test by ANOVA).

**Figure 5.** Enhancement of $I_{GABA}$ by compound **4** (■), compound 1 (•), compound 6 (♦) and compound **10** (▲) at a GABA $EC_{3-10}$ concentration through $\alpha_1\beta_2\gamma_{2S}$ receptors; each data point represents the mean ± S.E.M. from at least 3 oocytes and ≥ 2 oocyte batches (A). Representative currents for the $I_{GABA}$ modulation by compound **4** (**B**).

**Figure 6.** Effect of i.p injection of compound **4** at the indicated dose (mg/kg/bodyweight) on the performance of mice in the open field test described in Example 2. The bars indicate the distance traveled in the open field in 10 min. Data points represent means ± S.E. from at least 9 different mice. (*) indicates statistically significant ($p<0.05$) differences.

**Figure 7.** Comparison of the *in vivo* effect of compound **4** and compound **1** (actein) at a dose of 20 mg/kg/bodyweight on the performance of mice in the open field test described in Example 2. The bars indicate the distance travelled in the open field in 10 min. Data points represent means ± S.E. from at least 9 different mice. (*) indicates statistically significant ($p<0.05$) differences.

**Figure 8.** Effect of i.p injection of compound **4** at the indicated dose (mg/kg/bodyweight) on the performance of mice in the Light/Dark choice test described in Example 2. The bars indicate the time spent in the lit area (400 Lux). Data points represent means ± S.E. from at least 9 different mice. (*) indicates statistically significant ($p<0.05$) differences.

**Figure 9.** Effect of i.p injection of compound **4** at the indicated dose (mg/kg/bodyweight) on stress-induced hyperthermia, as described in Example 2. The bars indicate the body temperature increase upon stress. Data points represent means ± S.E. from at least 9 different mice. (*) indicates statistically significant ($p<0.05$) differences.

The invention will now be described by reference to the hollowing examples which are merely illustrative and are not to be construed as a limitation of the scope of the present invention.

## Examples

### Example 1: Bioactivity-guided isolation of cycloartane glycosides from Actaea *racemosa* and determination of $I_{GABA}$ Modulation of the isolated compounds

### General experimental procedures

All reagents were of analytical quality, organic solvents for isolation were distilled before use and water was nanopure water. Column chromatography was performed with Silica gel (40-63 μm particle size) (Merck, Germany) or with Sephadex LH-20 (Sigma Aldrich, USA). TLC was performed on silica gel 60 $F_{254}$ plates (VWR, Germany) using

chloroform-acetone-methanol (70:15:15, v/v/v) as mobile phase and vanillin-sulphuric acid (reagent A: 1 % vanillin in methanol, reagent B: 5 % sulphuric acid in methanol) for detection. Preparative LPLC was accomplished using a Merck Hitachi Pump L-7100 and Lichroprep RP-18 Silica gel (40-63 μm particle size) (Merck, Germany). Semi-preparative HPLC was carried out on a Dionex system (Dionex, USA) with a P580 pump, ASI-100 autosampler, UVD 170U detector, Gilson 206 fraction collector (Gilson Inc., USA) and a Phenomenex Synergi Max-RP column (250 x 10 mm, 10 μm particle size) (Phenomenex, Germany). Extracts, fractions and substances were analyzed by HP 1090, connected to an autosampler, column heater, DAD and Alltech ELSD 2000 instrument (Alltech Associates, USA) using a Phenomenex Synergi Max-RP column (150 x 4.6 mm, 4 μm particle size). MS analysis was carried out by direct injection using a Bruker Daltonics Esquire 3000$^{plus}$ instrument with atmospheric pressure chemical ionization (APCI). Nuclear magnetic resonance spectra were recorded on a Bruker DRX-300 instrument (Bruker, Germany).

**Planet material**

Dried and cut sub-aerial parts of *Actaea racemosa* L. were obtained from Mag. Kottas Heilkräuter, Austria (Rd. Cimicifugae Cs. EB 6, lot number: KLA50226).

**Extraction and isolation**

Dried rhizome (700 g) was grinded and extracted with 2 L of MeOH under reflux for 3 days and the solvent was evaporated under reduced pressure to afford 108 g of crude extract. The extract was then partitioned between diethyl ether and water for 10 times, and the diethyl ether layer was rewashed with water and concentrated to dryness, yielding a residue of 27 g. Diethyl ether extract (26 g) was subjected to silica gel column chromatography (25 x 10 cm) and eluted with a petroleum ether/acetone/ethyl acetate (1:1:1) yielding 8 yellow fractions (A1I-A1H). After the eluting solvent turned colourless 10 % methanol was added to the solvent mixture to collect fractions A1I-A1N. A1O was obtained by flushing the column with methanol. Fraction A1J (2.40 g) was chromatographed with silica gel column (100 x 3.5 cm) using n-heptane/ethyl acetate/methanol (10:10:0.5 to 10:10:3) in a gradient manner yielding 12 subfractions (A1J1-A1J12). Fraction A1J12 gave 74 mg of compound **1** and fraction A1J10 gave 121 mg of compound **3**. Fractions A1J8 and A1J6 were recrystallized to afford 23 mg of compound **2** and 57 mg of compound **4**. Fractions A1 K-A1 N were combined (4.45 g) and subjected to a silica gel column (45 x 5.5 cm) using a solvent system of dichloromethane-methanol (in a step gradient from 9:1 to 7:3) obtaining 6 subfractions (A1KN1-A1KN6). Fraction A1KN2 (1.65 g) was chromatographed with silica gel column (100 x 3.5 cm) using *n*-heptane/ethyl acetate/methanol (10:10:0.5 to 10:10:3) in a gradient manner yielding 7 subtractions (A1KN21-A1KN27). Subtraction A1KN23 afforded 26 mg of compound **7**. Also fraction A1KN4 (0.83 g) was subjected to Silica gel column chromatography using *n*-heptanelethyl acetate/methanol (10:10:1 to 10:10:3) giving 8 subfractions (A1 KN41-A1 KN48). Subfractions A1 KN42 and A1 KN43 were combined (204 mg) and subjected to LPLC using $H_2O$/ MeCN (in a gradient from 7:3 to 4:6 over 4 h) to obtain 10 mg of compound **5** and 54 mg of compound **6**. Fraction A1I (0.98 g) was subjected to Sephadex LH-20 column chromatography (80 x 2 cm) using dichloromethane-aceton (85:15) as solvent mixture yielding 9 subfractions (A1I1-A1I9). Subtraction A1I3 (180 mg) was separated with semi-preparative HPLC using $H_2O$/MeCN (50:50) yielding 23 mg of compound **8** and 9 mg of compound **9**. Subfraction A1I4 (96 mg) was also subjected to semi-preparative HPLC using $H_2O$/MeC (45:55) to give 5 mg of compound **11**. Semi-preparative HPLC of subfraction A1I5 (78 mg) using $H_2O$/MeCN (40:60) afforded 8 mg of compound **10**.

**Expression and functional characterization of GABA$_A$ receptors**

Preparation of stage V-VI oocytes from *Xenopus laevis*, synthesis of capped off run-off poly(A$^+$) cRNA transcripts from linearized cDNA templates (pCMV vector) was performed as described (Khom et al. 2000). Briefly, female *Xenopus laevis* (NASCO, USA) were anaesthetised by exposing them for 15 minutes to a 0.2 % solution of MS-222 (methane sulfonate salt of 3-aminobenzoic acid ethyl ester; Sandoz) before surgically removing parts of the ovaries. Follicle membranes from isolated oocytes were enzymatically digested with 2 mg/ml collagenase (Type 1A, Sigma). One day after isolation, the oocytes were injected with about 10-50 nl of DEPC- treated water (dimethyl pyrocarbonate, Sigma, Germany) containing the different cRNAs at a concentration of approximately 150 - 3000 ng/μl/subunit. The amount of cRNA was determined by means of a NanoDrop ND-1000 (Kisker-biotech, Steinfurt, Germany).

To ensure expression of the gamma-subunit, cRNAs were mixed in a ratio of 1:1:10 (Boileau et al., 2002). Oocytes were stored at 18°C in ND96 solution (Methfessel et al., 1986). Electrophysiological experiments were done using the two-microelectrode voyage-clamp method at a holding potential of -70 mV making use of a TURBO TEC 01C amplifier (npi electronic, Tamm, Germany) and an Axon Digidata 1322A interface (Molecular Devices, Sunnyvale, CA). Using pCLAMP v.9.2 data acquisition was carried out. The bath solution contained 90 mM NaCl, 1 mM KCl, 1 mM $MgCl_2 \cdot 6H_2O$, 1 mM $CaCl_2$ and 5 mM HEPES (pH 7.4). Microelectrodes were filled with 2M KCl and had resistances

between 1 and 3 MΩ (Khom et al., 2007).

**Perfusion system**

GABA and compound (extract, fraction or pure compound) were applied by means of fast perfusion system (see Baburin et al., 2006 for detail Drug or control solutions were applied by means of a TECAN Miniprep 60 permitting automation of the experiments. Oocytes with maximal current amplitudes >3 μA were discarded to exclude voltage-clamp errors (Khom et al., 2007).

**Analysing concentration-response curves**

Stimulation of chloride currents by modulators of the GABAA receptor was measured at a GABA concentration eliciting between 3 and 10% of the maximal current amplitude (EC3-10). The EC3-10 was determined at the beginning of each experiment. Enhancement of the chloride current was defined as $(I_{(GABA+Comp)}/I_{GABA}) - 1$, where $I_{(GABA+Comp)}$ is the current response in the presence of a given compound (extract, fraction or pure compound) and $I_{GABA}$ is the control GABA current. To measure the sensitivity of the $GABA_A$ receptor for a given compound, it was applied for an equilibration period of 1 minute before applying GABA ($EC_{1-10}$). Concentration-response curves were generated and the data were fitted by non-iinear regression analysis using Origin software (OriginLab Corporation, USA). Data were fitted to the equation:

$$\frac{1}{1+\left(\frac{EC_{50}}{[Comp]}\right)^{n_H}},$$

where $n_H$ is the Hill coefficient. Each data point represents the mean ± S.E. from at least 4 oocytes and ≥ 2 oocyte batches. Statistical significance was calculated using paired Student *t*-test with a confidence interval of $p < 0.05$.

**Bioactivity-guided isolation**

As described above, dried and cut sub-aerial parts of *A. racemosa* were grinded and extracted with methanol. The resulting crude extract (A0) was subsequently partitioned between water and organic solvents of increasing polarity giving diethyl ether (A1), ethyl acetate (A2) and 1-butanol (A3) fractions. Obtained extracts and the remaining aqueous layer (A4) were tested for their ability to modulate $I_{GABA}$ at a concentration of 100 μg/mL. The results are shown in Figure 2 and Table 1 below. To analyse the modulation of $I_{GABA}$ by black cohosh extracts and isolated compounds, $GABA_A$ receptors composed of $\alpha_1$, $\beta_2$ and $\gamma_{2s}$ subunits were expressed in *Xenopus* oocytes. Stimulation of $I_{GABA}$ was analysed by means of the 2-microelectrode voyage-clamp technique. These and all subsequent experiments were carried out at a GABA $EC_{3-10}$ concentration.

**Table 1:** Stimulation of $I_{GABA}$ by the indicated extracts (100 μg/ml) at a GABA $EC_{3-10}$ concentration.

| Extract | Stimulation of $I_{GABA}$ (%) | number of experiments (n) |
|---|---|---|
| **A0** | 162 ± 19 | 5 |
| **A1** | 270 ± 53 | 4 |
| **A2** | 113 ± 14 | 4 |
| **A3** | 21 ± 18 | 3 |
| **A4** | -1 ± 5 | 3 |

As shown in Table 1 and Figure 2, the crude methanol extract (A0) significantly enhanced $I_{GABA}$ (162 ± 19%, n=5). Partitioning the methanol extract with diethyl ether resulted in a significantly stronger potentiation of $I_{GABA}$ (270 ± 53%. n = 4, p< 0.05), indicating that the activity is attributable to the more apolar constituents of the crude extract. HPLC-UV and HPLC-ELSD analysis of A1 showed a high content of weak UV-absorbing compounds, which were assumed to

be cycloartane glycosides. HPLC chromatograms of extract A2 were similar to those of A1, apart from a higher ratio of UV-absorbing substances. Stimulation of $I_{GABA}$ by extract A2 was less pronounced compared to A1 (113±14, n=4). No significant stimulation of $I_{GABA}$ was induced by extracts A3 and A4, suggesting that the triterpenoids were quantitatively extracted from the aqueous layer.

Subsequently A1 was separated by silica gel column chromatography. A very apolar solvent mixture was chosen to initially elute oily and resinous constituents from the column, while keeping the glycosides adsorbed onto the material, resulting in 8 fractions (A1A-A1H). Thereupon methanol was added to the solvent mixture and triterpenoids were eluted yielding another 6 fractions (A1I-A1N): Finally, the column was flushed with methanol A1O). The effects of these fractions on $I_{GABA}$ ($EC_{3-10}$) were analysed on *xenopus* oocytes at a concentration of 100 μg/mL. The results are shown in Figure 3 and Table 2 below.

**Table 2:** Stimulation of $I_{GABA}$ in % by 100 μg/ml of the indicated fractions at a GABA $EC_{3-10}$ concentration.

| Fraction | Stimulation of $I_{GABA}$ (%) | number of experiments (n) |
|---|---|---|
| **A1A** | 76 ± 10 | 4 |
| **A1B** | 53.2 ± 11 | 4 |
| **A1C** | 59 ± 15 | 4 |
| **A1D** | -5 ± 5 | 3 |
| **A1E** | 2 ± 10 | 4 |
| **A1F** | 90 ± 35 | 4 |
| **A1G** | 138 ± 40 | 4 |
| **A1H** | 200 ± 63 | 3 |
| **A1I** | 536 ± 48 | 4 |
| **A1J** | 569 ± 38 | 4 |
| **A1K** | 430 ± 42 | 4 |
| **A1L** | 525 ± 4 | 4 |
| **A1M** | 557 ± 46 | 3 |
| **A1N** | 581 ± 112 | 3 |
| **A1O** | 199 ± 56 | 3 |

While fractions A1A-A1H, consisting of dark brown viscous material, displayed significant but relatively weak (A1A-A1C) potentiation of $I_{GABA}$ or no effect (A1D-A1E), fractions A1I-A1N showed strong $I_{GABA}$ enhancement, as shown in Table 2, suggesting that the active components could be enriched. These latter fractions appeared as white to pale yellow powders except fraction A1I, which was ochre. Fraction A1O was a brown powder and found to contain mainly polar substances. HPLC-UV/ELSD analysis of active fractions A1I-A1N showed that the content of UV-absorbing substances in these fractions was reduced to a minimum and triterpene glycosides were highly enriched. Thus, further work concentrated on the isolation of the main compounds out of fractions A1I-A1N and on the subsequent characterization of $GABA_A$ receptor modulation by these compounds.

Fraction A1J showed 4 compounds, which were all isolated by silica gel column chromatography and identified as actein **(1)** (Kusano et al., 1998), 26-deoxyactein **(2)** (Chen et al., 2002), 23-epi-26-deoxyactein **(3)** (Chen et al., 2002) and 23-O-acetylshengmanol-3-O-β-D-xylopyranoside **(4)** (Watanabe et al., 2002), with **1** and **3** as major constituents. Fractions A1K-A1N did not differ in their constituent pattern, as compounds **4-7** were apparent in all fractions, with decreasing content **of 4 and 7** and increasing amounts of compounds **5** and **6.** These 4 fractions were combined and repeatedly chromatographed to obtain cimiracemoside F **(5),** cimigenol-3-O-β-D-xylopyranoside (6) and cimiracemoside D (**7**) (Shao et al)., 2000). Fraction A1I showed 3 major components **(8-10),** which were all isolated (along with one minor constituent **11)** using different chromatographic methods. Structure elucidation resulted in the identification of 25-O-acetylcimigenol-3-O-α-L-arabinopyranoside **(8)** (Shao et al., 2000), 25-O-anhydrocimigenol-3-O-α-L-arabinopyranoside **(9),** 25-O-anhydrocimigenol-3-O-β-D-xylopyranoside **(10)** and cimiracemoside K **(11)** (Chen et al., 2002). All isolated substances were analysed for $GABA_A$ receptor modulation, whereas significant enhancement of $I_{GABA}$ could be determined for compounds **1, 4, 6** and **8.**

**$I_{GABA}$ Modulation by compounds 1, 4, 6, and 8**

As shown in Figure 4, 100 μM of compounds **1, 4, 6, and 8** significantly ($p<0.05$) enhanced $I_{GABA}$ by more than 150%, when co applied with GABA ($EC_{3-0}$). The strongest effect was observed for compound **4** (23-O-acetylshengmanol-3-O-β-D-xylopyranoside; 100 μM) enhancing $I_{GABA}$ by 1692 ± 201 %, while compound **1** (actein), compound **6** (cimigenol-3-O-β-D-xylopyranoside) and compound **8** (25-O-acetylcimigenol-3-O-α-L-arabinopyranoside) were significantly less efficient (range of $I_{GABA}$ enhancement at 100 μM: 256 ± 40 for compound **6-**378 ± 64% for compound **1).** Compound **4** thus induced an about 10-times stronger $I_{GABA}$ enhancement than the other compounds.

Except compound **4,** none of these compounds induced chloride currents when applied in the absence of GABA. These chloride currents induced by compound **4** were, however, relatively small and did not exceed 1% of $I_{GABA-max}$ (induced by application of 1mM GABA; data not shown).

To elucidate whether cleavage of the pentose moiety affects $I_{GABA}$ modulation the active compounds **1, 4** and **6** were hydrolysed into 12-acetylacteol **(1a),** 23-O-acetylshengmanol **(4a)** and cimigenol **(6a).** Accordingly, for cleavage of β-D-xylose from position C-3, compounds **1** (35 mg), **4** (26 mg) and **6** (24 mg) were each suspended in 3.5 ml sodium acetate buffer (pH 4.3) containing 140 mg of naringinase and incubated at 20 °C for 72 h under constant stirring. The reaction process was controlled every 24 h by TLC. The aglycones were extracted from the reaction mixtures using *n*-hexane (10 ml, 5 times). The *n*-hexane layers were combined, rewashed with nanopure water and evaporated to dryness, giving yields of 23 mg of actein **(1a)** 14 mg of 23-O-acetylshengmanol **(4a)** and 12 mg of cimigenol **(6a).** Identity of the aglycones was confirmed by NMR spectroscopy. $I_{GABA}$ modulation by the resulting aglycones was significantly less pronounced. This loss of activity was most evident for compound **4** (from 1692 ± 201 to 64 ± 14) and less pronounced for compounds **1, 6** and **8,** as shown in Figure 4 and Table 3 below.

**Table 3:** Stimulation of $I_{GABA}$ (GABA $EC_{3-10}$ concentrations) at 100 μM of the indicated compound.

| Compound | Stimulation of $I_{GABA}$ (%) | number of experiments (n) |
|---|---|---|
| **1** | 305 ± 20 | 7 |
| **2** | 10 ± 6 | 3 |
| **3** | 10 ± 4 | 3 |
| **4** | 1692 ± 201 | 6 |
| **5** | 5 ± 2 | 4 |
| **6** | 189 ± 14 | 6 |
| **7** | 35 ± 9 | 3 |
| **8** | 271 ± 39 | 5 |
| **9** | -84 ± 3 | 3 |
| **10** | -76 ± 7 | 3 |
| **11** | -1 ± 26 | 3 |
| | | |
| **1a** | 126 ± 29 | 4 |
| **4a** | 64 ± 14 | 4 |
| **6a** | 104 ± 15 | 4 |

Compounds **1, 4, 6,** and **8** dose-dependently enhanced $I_{GABA}$ with comparable potencies ($EC_{50}$ ranged from 26 ± 7 (10) to 36 ± **14** (1), $p>0.05$). Compound **4** induced the strongest $I_{GABA}$ potentiation at 300 μM (1947 ± 185 %), while compounds **1, 6** and **8** were considerably less efficient ($I_{GABA}$ enhancement < 300%), as shown in Figure 5 and Table 4 below.

**Table 4:** Summary of efficiencies and potencies of isolated compounds **1, 4, 6** and **8**.

| Compound | $EC_{50}$(μM) | Maximal stimulation of $I_{GABA}$ | Number of experiments (n) |
|---|---|---|---|
| **1** | 36 ± 14 | 378 ± 64 | 7 |
| **4** | 27 ± 8 | 1947 ± 185 | 6 |

(continued)

| Compound | $EC_{50}$(μM) | Maximal stimulation of $I_{GABA}$ | Number of experiments (n) |
|---|---|---|---|
| **6** | 28 ± 17 | 256 ± 40 | 6 |
| **8** | 26 ± 7 | 289 ± 45 | 5 |

**Example 2: *In vivo* analysis of 23-O-acetylshengmanol-3-O-β-D-xylopyranosid (compound 4)**

The *in vivo* properties of 23-O-acetylshengmanol-3-O-β-D-xytopyranoside (compound **4**) were analysed. Male mice either treated with vehicle control) or different doses of compound **4** (0.2 mg, 0.6 mg, 2 mg, 6 mg, 20 mg or 60 mg per kg bodyweight) were tested in paradigms for providing information about sedative and/or anxiolytic effects. As described in the following, compound **4** has been demonstrated to be a compound inducing strong sedative effects with concomitant anxiolytic activity.

**Methods**

All procedures involving animals were approved by the Austrian Animal Experimentation Ethics Board in compliance with the European convention for the protection of vertebrate animals used for experimental and other scientific purposes ETS no.: 123. Every effort was taken to minimize the number of animals used.

Male mice (C57Bl/6N) were obtained from Charles River Laboratories (Germany).

For maintenance mice were group housed with free access to food and water. Temperature was fixed to 23°C and 60% humidity with a 12 h light-dark cycle (lights on 0700-1900 hours). Mice at 3-8 months age were tested in all experiments (Khom et al., 2010).

**Behavioral Testing**

Mice were transferred to the testing facility 24 h before commencement of experiments. Tests were performed between 0900 and 1300 hours. All tests were evaluated by an experimenter blinded to the administered compound, 30 min prior to testing animals were injected either with saline (0.9% NaCl-solution) as a control or with 23-O-acetylshengmanol-3-O-β-D-xylopyranosid (i.e., compound **4)** and actein (i.e., compound **1**), respectively (50 mg/250 μL stock prepared in 100% DMSO) at the indicated doses (0.2 mg/kg, 0.6 mg/kg, 2 mg/kg, 6 mg/kg, 20 mg/kg and 60 mg/kg bodyweight). All injection solutions contained 3% Polysorbat 80 and 10% of DMSO.

**pen Field Test (OF)**

To determine explorative behaviour mice treated (n=9 for all doses) with compound **4** and control (n=11 mice were studied in the OF.

Open field behavior was tested over 10 min in a 50x50 cm box equipped with infrared rearing detection. Illumination was set to 150 lux. Explorative behavior was recorded and analyzed using the ActiMot 2 equipment (infrared beams) and software (TSE-systems, Bad Homburg, Germany). Arenas were subdivided into border (up to 8 cm from wall), center (20x20 cm, ie 16% of total area), and intermediate area according to the recommendations of EMPRESS (European Mouse Phenotyping Resource of Standardised Screens; http://empress.har.mrc.ac.uk).

As shown in Figure 6, doses ≥2 mg/kg/bodyweight significantly (p<0.05) decreased ambulation in all areas of the OF as well as the overall ambulation compared to control animals, at doses ≥20 mg/kg mice covered an approximately 50% reduced distance compared to control littermates (38.2 ± 1.8 m control vs. 22.6 ± 2.8 m 20 mg/kg/bodyweight compound **4**).

Administration of 20 mg/kg/bodyweight compound **1** significantly (p<0.05) reduced the total distance travelled compared to control animals (38.2 ± 1.8 m control vs. 30.6 ± 2.1 m compound **1**), however, this decrease was less pronounced compared to the effect induced by the same dose of compound **4** (30.6 ± 2.1 m compound **1** vs. 22.6 ± 2.8 m compound **4**), as shown in Figure 7.

A reduction in motoractivity is commonly assumed to be a reliable parameter to assess the sedative properties of a compound. Both compounds **1** and **4** significantly decreased the distance traveled (= motoractivity) in the OF, however, this effect was more pronounced for compound **4**, indicating that this compound induces stronger sedation *in vivo*. Compound **4** is thus particularly useful as a sedative agent.

### Light/Dark Choice Test (LDT)

Explorative behavior in a brightly lit area (400 lux) was investigated by insertion of a black box into the open field arena, covering one third of the space. Time spent and distance travelled was measured over a 10 min period in the open area. One small field directly at the entrance to the black box was assigned as transition zone. To reach the larger compartment assigned as open area, the mouse had to leave the dark area completely. Behaviour was recorded and analyzed by means of the ActiMot2 system and software (TSE-systems, Bad Homburg, Germany).

In the LDT, a dose-dependent increase of the time spent in the brightly lit area was observed for mice injected with compound **4,** as shown in Figure 8, without significant effects on the distance travelled in the lit area (data not shown). This effect was, however, only significant ($p < 0.05$) for a dose of 0.6 mg/kg/bodyweight (151 $\pm$ 24 s control vs. 265 $\pm$ 52 s 0.6 mg/kg/bodyweight compound **4).**

Higher doses were not tested due to the observed reduced activity in the OF (shown in Figure 6). Transitions from the dark compartment to the lit area did not differ in the vehicle group or the compound **4**-treated littermates (data not shown).

### Stress-induced Hyperthermia Test (SIH)

A temperature probe, lubricated with petrolatum, was inserted into the rectum of the mouse for a depth of up to 2 cm. The temperature probe remained in the animal till a stable temperature was reached (maximum 10 s).

Prior to i.p injection of compound or saline the basal body temperature was determined. 3h after i.p injection, temperature was measured twice by means of a TH-5 Thermalert Monitoring Thermometer combined with a RET-3 rectal probe for mice.

Temperature measurement was repeated after 10 min and the rise in temperature between the first and second measurement was considered as stress-induced hyperthermia (referred as $\Delta$T).

In the SIH, an increase of $\Delta$T of 1.2 $\pm$ 0.1° (n=9) was observed for control mice. Administration of compound **4** induced a significantly ($p<0.05$) smaller increase, as shown in Figure 9. For mice treated with a dose of 6 mg/kg/ bodyweight no significant increase of $\Delta$ (0.1 $\pm$ 0.1°; n=9; $p<0.05$) was observed any more.

Compound **4** caused a significant increase of the time spent in the brightly lit area in the light/dark-choice test and a significantly smaller increase in AT compared to the control in the stress-induced hyperthermia test, respectively, as shown in Figures 8 and 9. Such behaviour is commonly associated with an anxiolytic-like activity of a compound. Thus, in both tests clearly a reduction in anxiety-related behaviour was observed, indicating that compound **4** exerts anxiolytic properties *in vivo.* Compound **4** is thus particularly useful as an anxiolytic agent.

## Claims

1. A compound of formula (I) or an enantiomer or diastereomer thereof

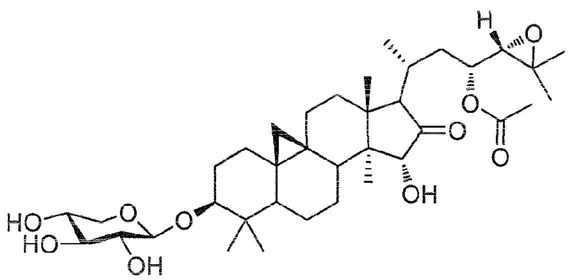

(I)

or a pharmaceutically acceptable salt, solvate or prodrug thereof
for use as an anxiolytic agent, a sedative agent, a hypnotic agent, an anticonvulsant agent, an analgesic agent, an anesthetic agent, an antiepileptic agent, and/or a muscle relaxant.

2. A pharmaceutical composition comprising the compound of claim 1 and a pharmaceutically acceptable excipient for use as an anxiolytic agent, a sedative agent,

a hypnotic agent, an anticonvulsant agent, an analgesic agent, an anesthetic agent, an antiepileptic agent, and/or a muscle relaxant.

3. The compound of claim 1 or the pharmaceutical composition of claim 2 for use in treating or preventing insomnia, anxiety, pain, mood disorders, panic disorders, epilepsy, schizophrenia, disorders/symptoms connected to or associated with alcohol and/or substance withdrawal or abuse, or climacteric or postmenopausal symptoms.

4. The compound of dam 1 or the pharmaceutical composition of claim 2 for use in treating or preventing anxiety, anxiety disorders, generalized or substance-induced anxiety disorder, panic disorder with or without agoraphobia, agoraphobia without history of panic disorder, phobias, social phobias, obsessive-compulsive disorder, Down Syndrome, sleep disorders, cognitive disorders, seizure disorders, epilepsy, pain, depression or bipolar disorders, single-episode or recurrent major depressive disorder, dysthymic disorder, bipolar I manic disorder, bipolar II manic disorder, neuroses, schizophrenia, attention deficit hyperactivity disorder, disorders of circadian rhythm, overdose with benzodiazepine drugs, disorders/conditions including the impairment of recent memory and/or remote memory and/or alertness, Huntington's Chorea, muscular spasms and rigidity, withdrawal symptoms, Alzheimer's disease, Parkinson's disease, stress disorders, or post-traumatic and/or acute stress disorder.

5. A method of treating or preventing a disease, disorder or condition, the method comprising the administration of the compound of claim 1 or the pharmaceutical composition of claim 2 to a subject in need of such a treatment or prevention, wherein said disease, disorder or condition is selected from insomnia, anxiety, pain, mood disorders, panic disorders, epilepsy, schizophrenia, disorders/symptoms connected to or associated with alcohol and/or substance withdrawal or abuse, or climacteric or postmenopausal symptoms.

6. A method of treating or preventing a disease, disorder or condition, the method comprising the administration of the compound of claim 1 or the pharmaceutical composition of claim 2 to a subject in need of such a treatment or prevention, wherein said disease, disorder or condition is selected from anxiety, anxiety disorders, generalized or substance-induced anxiety disorder, panic disorder with or without agoraphobia, agoraphobia without history of panic disorder, phobias, social phobias, obsessive-compulsive disorder, Down Syndrome, sleep disorders, cognitive disorders, seizure disorders, epilepsy, pain, depression or bipolar disorders, single-episode or recurrent major depressive disorder, dysthymic disorder, bipolar I manic disorder, bipolar II manic disorder, neuroses, schizophrenia, attention deficit hyperactivity disorder, disorders of circadian rhythm, overdose with benzodiazepine drugs, disorders/conditions including the impairment of recent memory and/or remote memory and/or alertness, Huntington's Chorea, muscular spasms and rigidity, withdrawal symptoms, Alzheimer's disease, Parkinson's disease, stress disorders, or post-traumatic and/or acute stress disorder.

7. The compound of claim 1, 3 or 4 or the pharmaceutical composition of any of claims 2 to 4 or the method of claim 5 or 6, whereby the compound or the pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route.

8. The method of any of claims 5 to 7, wherein the subject is a human.

Fig. 1

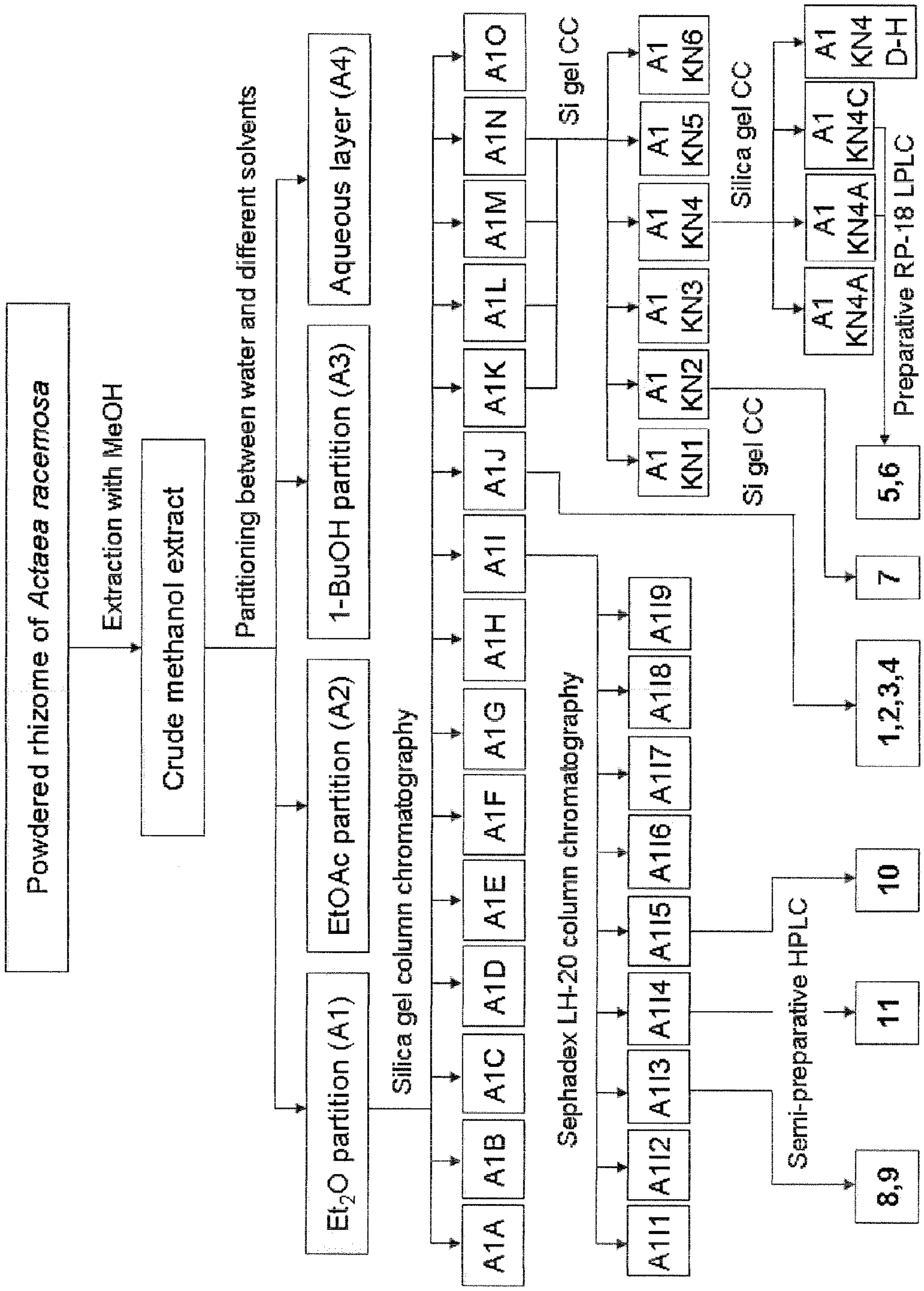

Powdered rhizome of *Actaea racemosa*
Extraction with MeOH
Crude methanol extract
Partitioning between water and different solvents
$Et_2O$ partition (A1)
EtOAc partition (A2)
1-BuOH partition (A3)
Aqueous layer (A4)
Silica gel column chromatography
A1A
A1B
A1C
A1D
A1E
A1F
A1G
A1H
A1I
A1J
A1K
A1L
A1M
A1N
A1O
Sephadex LH-20 column chromatography
A1I1
A1I2
A1I3
A1I4
A1I5
A1I6
A1I7
A1I8
A1I9
Semi-preparative HPLC
8,9
11
10
Si gel CC
1,2,3,4
Si gel CC
A1 KN1
A1 KN2
A1 KN3
A1 KN4
A1 KN5
A1 KN6
7
Silica gel CC
A1 KN4A
A1 KN4A
A1 KN4C
A1 KN4 D-H
Preparative RP-18 LPLC
5,6

Fig. 2

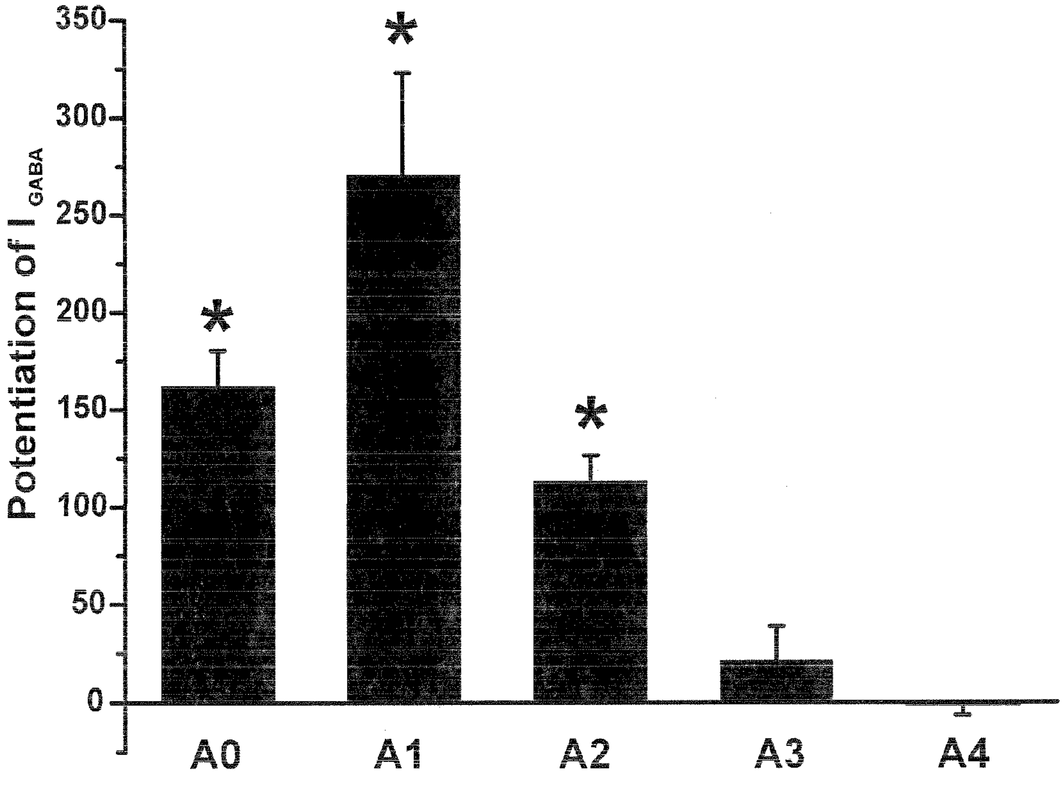
350
300
250
200
150
100
50
0
Potentiation of I GABA
*
*
*
A0
A1
A2
A3
A4

Fig. 3

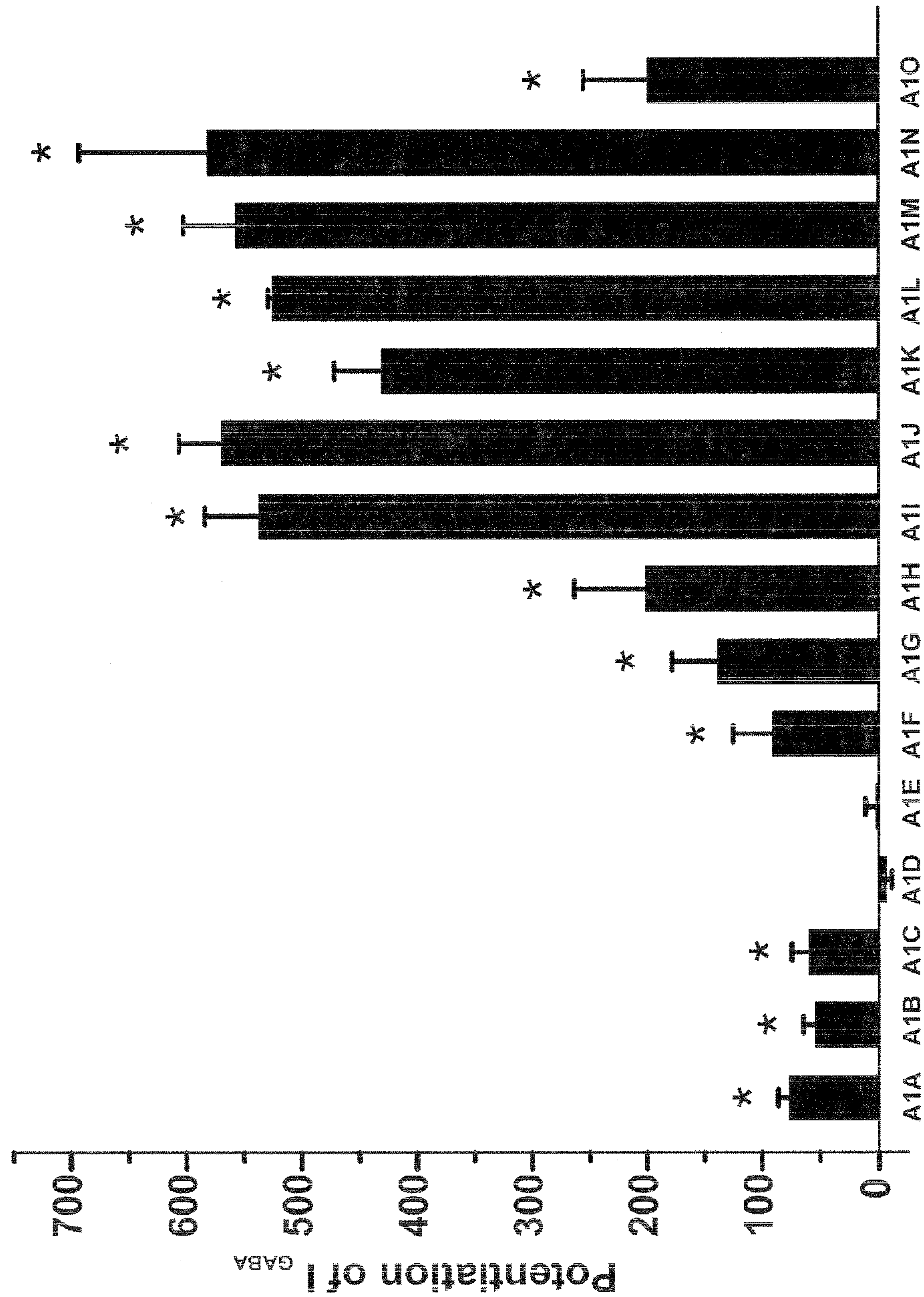
Potentiation of $I_{GABA}$
0
100
200
300
400
500
600
700
A1A
A1B
A1C
A1D
A1E
A1F
A1G
A1H
A1I
A1J
A1K
A1L
A1M
A1N
A1O

Fig. 4

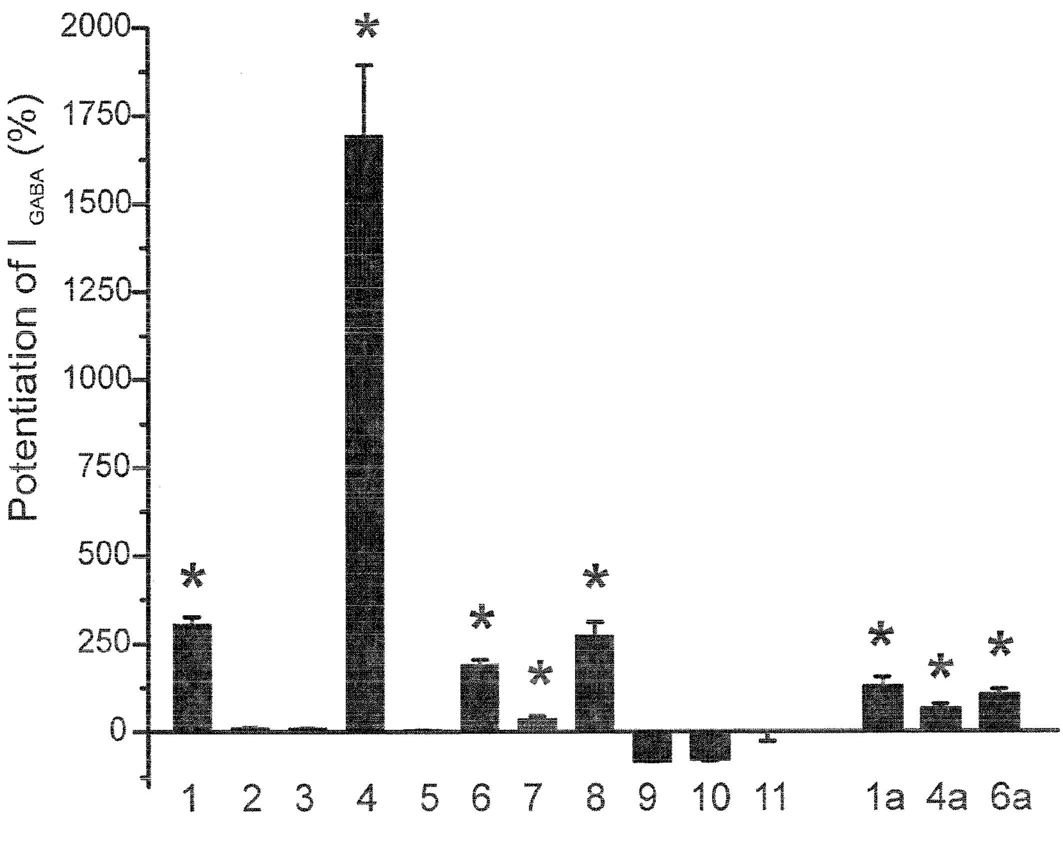
2000
1750
1500
1250
1000
750
500
250
0
Potentiation of I GABA (%)
1
2
3
4
5
6
7
8
9
10
11
1a
4a
6a
*

Fig. 5

**A**

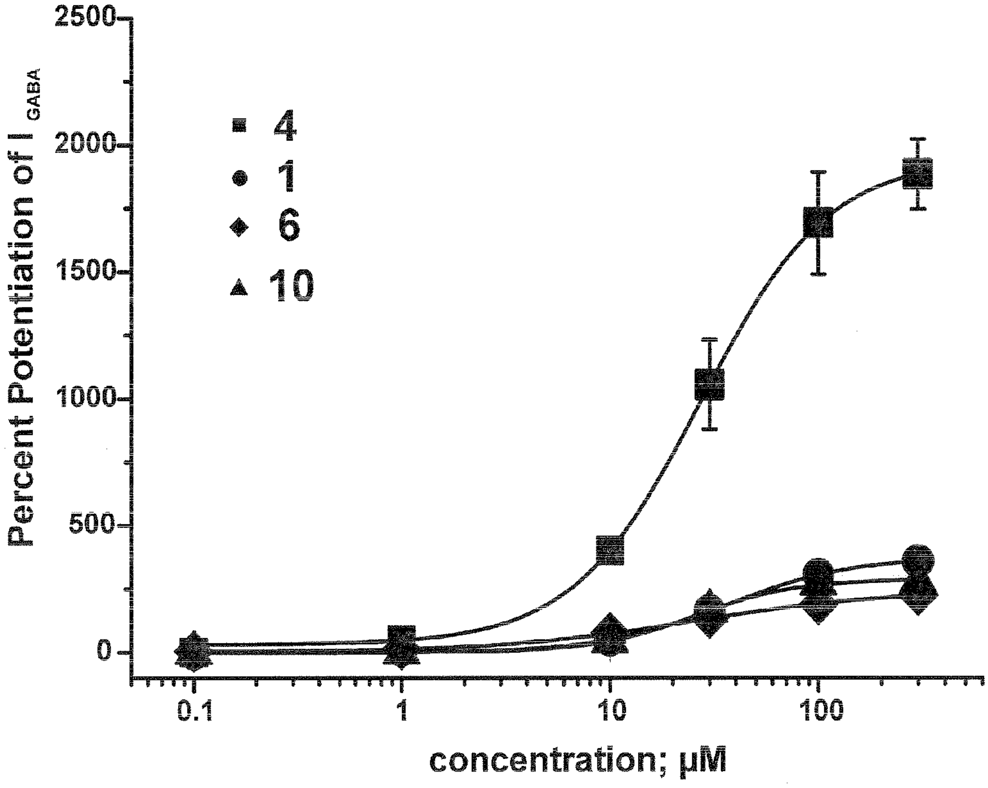
2500
2000
1500
1000
500
0
Percent Potentiation of I GABA
4
1
6
10
0.1
1
10
100
concentration; µM

Fig. 5 (cont.)

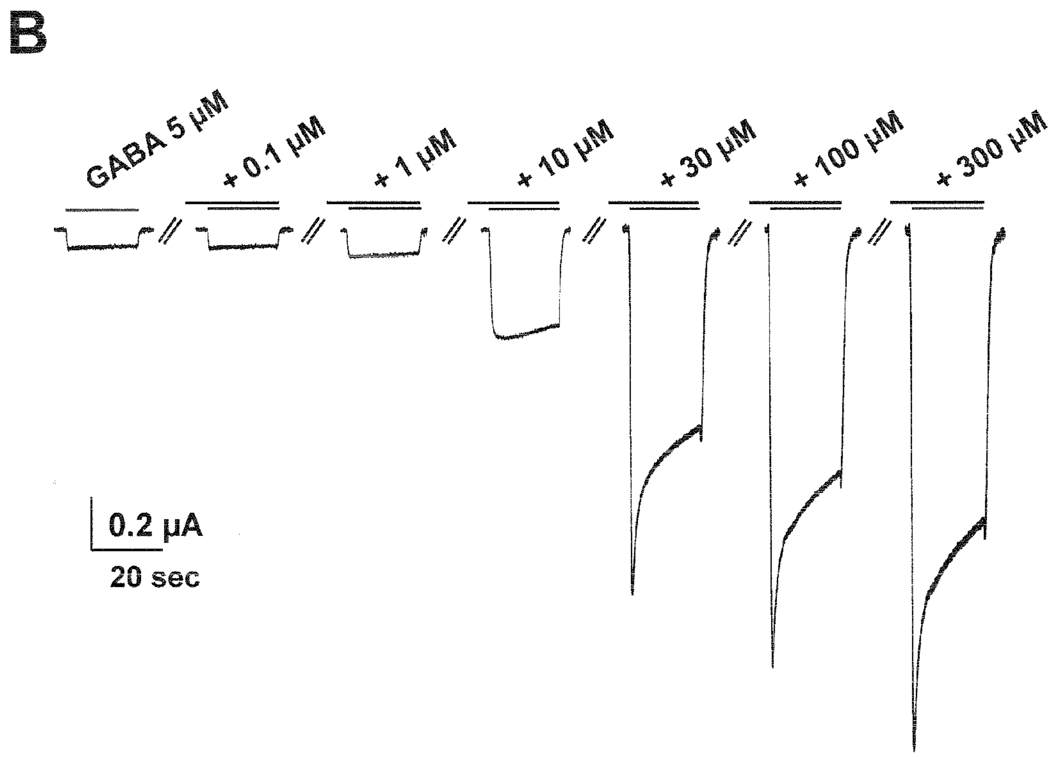

Fig. 6

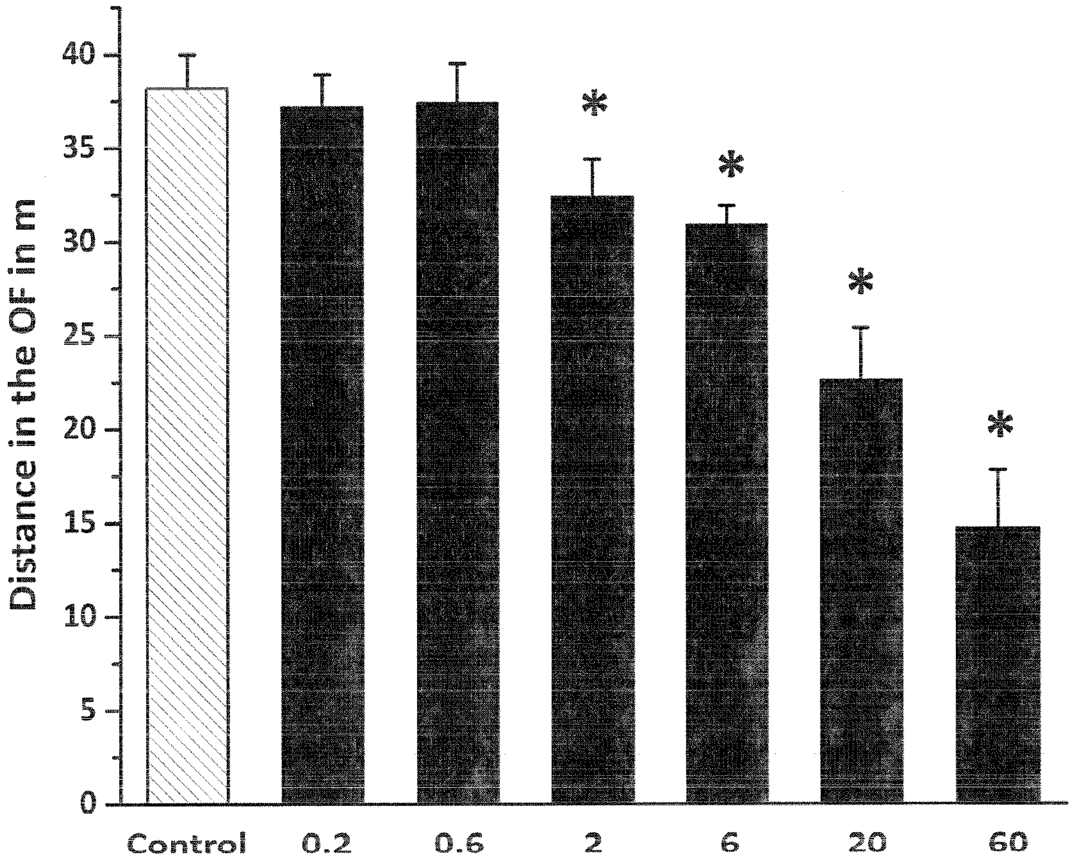
Distance in the OF in m
0
5
10
15
20
25
30
35
40
*
*
*
*
Control
0.2
0.6
2
6
20
60

Fig. 7

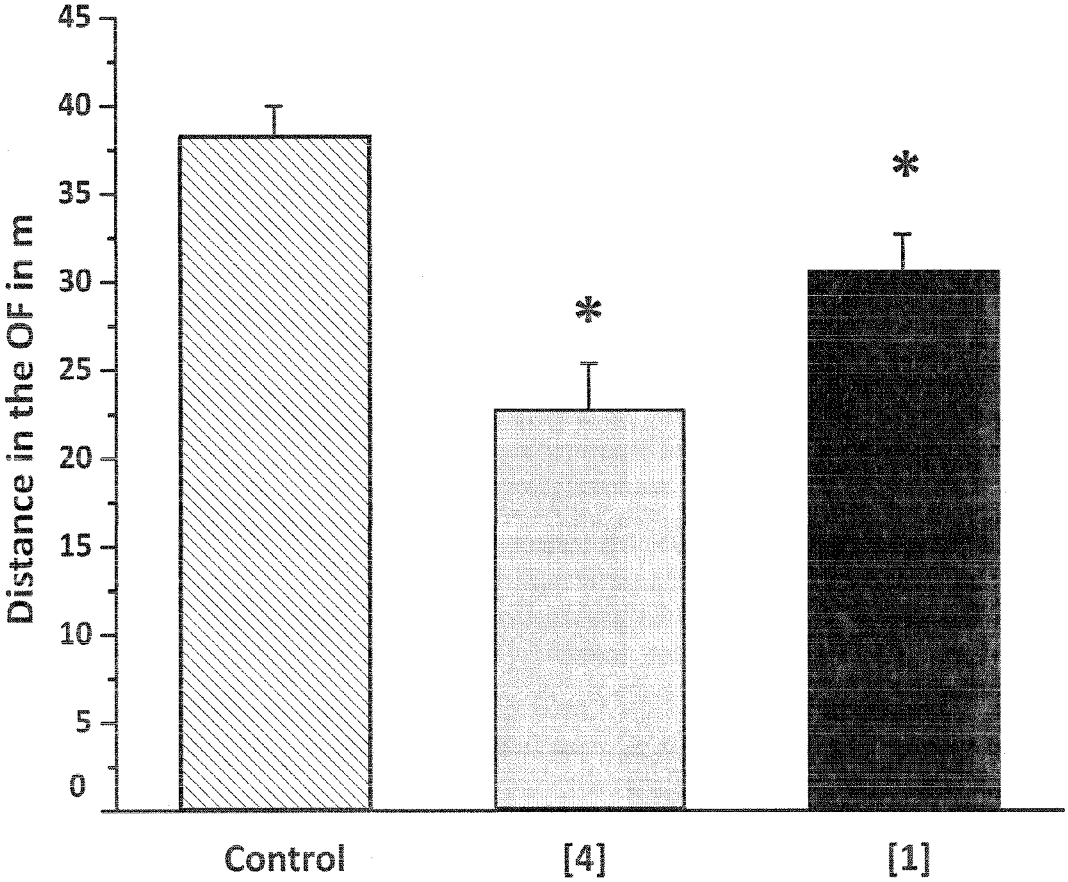

Fig. 8

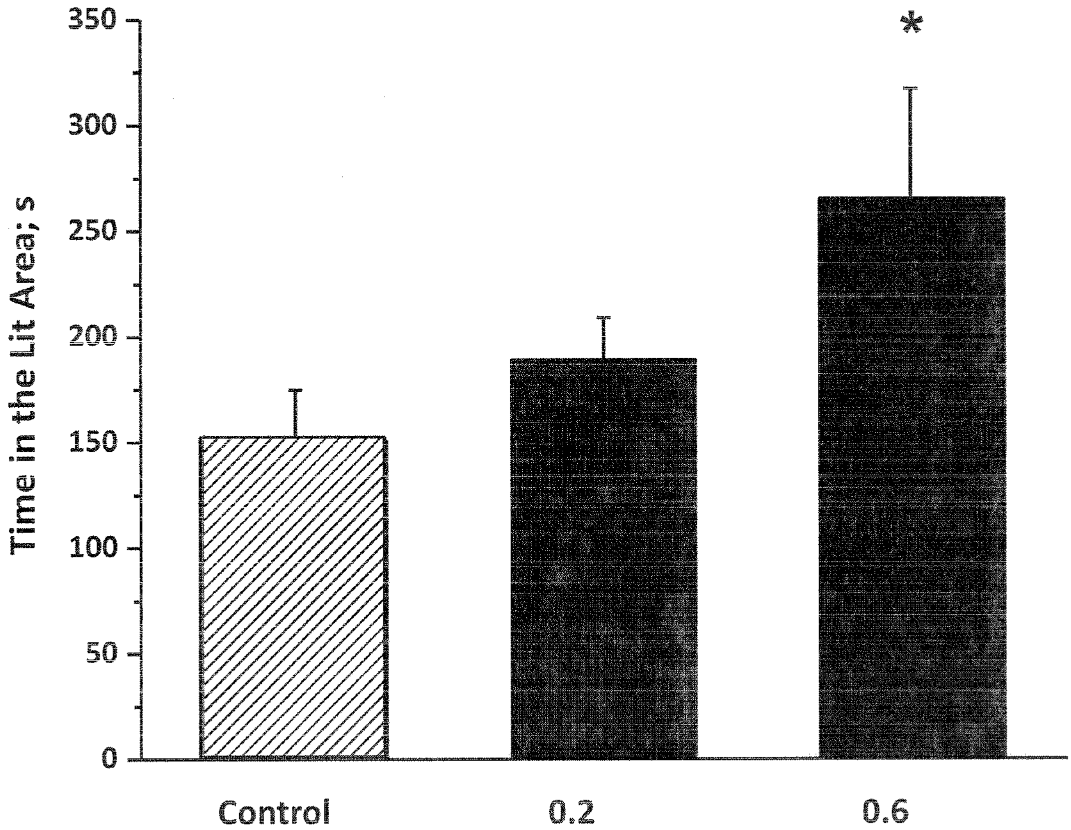

Fig. 9

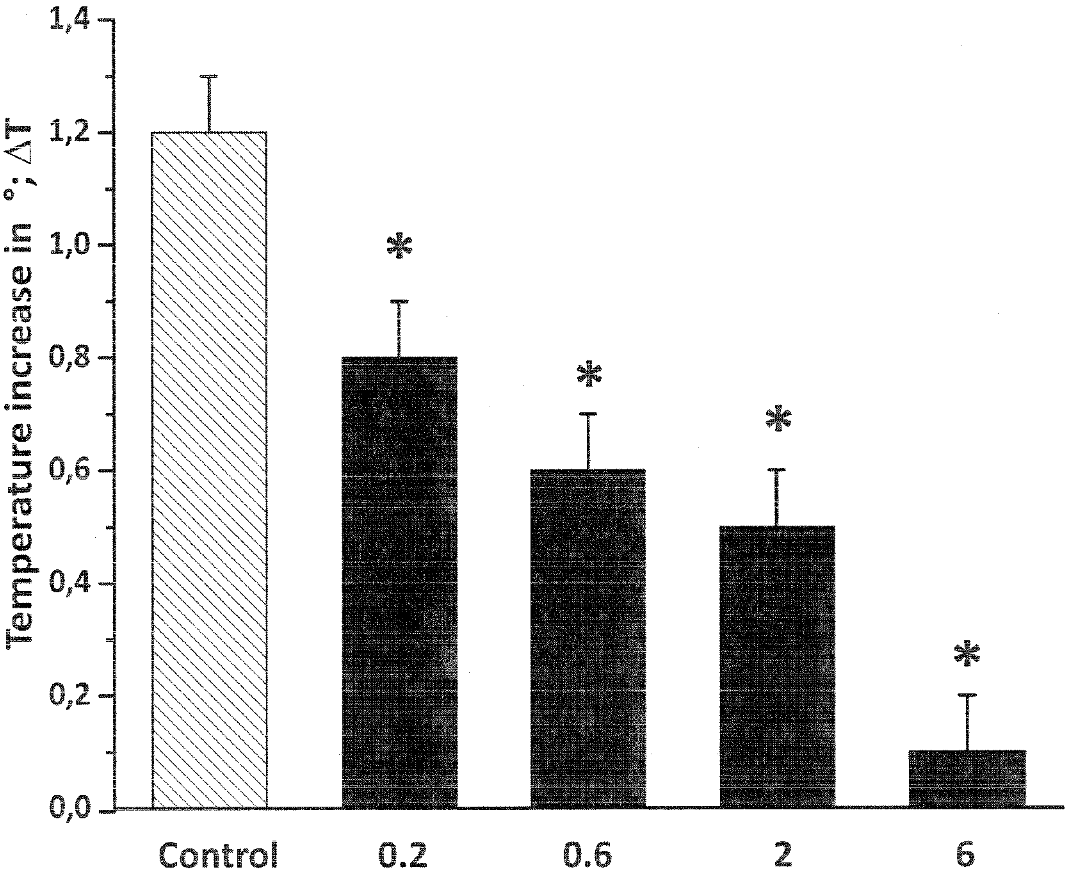
Temperature increase in °; ΔT
1,4
1,2
1,0
0,8
0,6
0,4
0,2
0,0
*
*
*
*
Control
0.2
0.6
2
6

Europäisches Patentamt
European Patent Office
Office européen des brevets

# EUROPEAN SEARCH REPORT

Application Number
EP 10 18 7121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE BIOSIS [Online] BIOSCIENCES INFORMATION SERVICE, PHILADELPHIA, PA, US; 1980, SHIBATA M ET AL: "PHARMACOLOGICAL STUDIES ON THE CHINESE CRUDE DRUG SHOMA 3. CENTRAL DEPRESSANT AND ANTI SPASMODIC ACTIONS OF CIMICIFUGA-RHIZOMA CIMICIFUGA-SIMPLEX", XP002620984, Database accession no. PREV198171083026 * abstract * & YAKUGAKU ZASSHI, vol. 100, no. 11, 1980, pages 1143-1150, ISSN: 0031-6903 ----- | 2-8 | INV. A61K31/704 A61P21/02 A61P29/00 A61P25/22 A61P25/08 A61P25/20 |
| X | DATABASE MEDLINE [Online] US NATIONAL LIBRARY OF MEDICINE (NLM), BETHESDA, MD, US; December 2007 (2007-12), CAO LI ET AL: "[Comparison of activities of various species of Rhizoma Cimicifugae and their honey processed products].", XP002620985, Database accession no. NLM18422192 * abstract * & ZHONG YAO CAI = ZHONGYAOCAI = JOURNAL OF CHINESE MEDICINAL MATERIALS DEC 2007 LNKD- PUBMED:18422192, vol. 30, no. 12, December 2007 (2007-12), pages 1561-1563, ISSN: 1001-4454 ----- | 2-8 | TECHNICAL FIELDS SEARCHED (IPC) A61K A61P |
| | -/-- | | |

The present search report has been drawn up for all claims

1

| Place of search | Date of completion of the search | Examiner |
|---|---|---|
| Munich | 8 February 2011 | Büttner, Ulf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 7121

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | KIM S J ET AL: "Inhibitory effects of cimicifugae rhizoma extracts on histamine, bradykinin and COX-2 mediated inflammatory actions.", PHYTOTHERAPY RESEARCH : PTR DEC 2000 LNKD-PUBMED:11113994, vol. 14, no. 8, December 2000 (2000-12), pages 596-600, XP002620986, ISSN: 0951-418X * page 599, last paragraph * ----- | 2-8 | |
| X | DATABASE WPI Week 200943 Thomson Scientific, London, GB; AN 2009-K30512 XP002620987, & CN 101 444 620 A (SUN H) 3 June 2009 (2009-06-03) * abstract * ----- | 2-8 | |
| X | SCHMID DIETHART ET AL: "Inhibition of inducible nitric oxide synthesis by Cimicifuga racemosa (Actaea racemosa, black cohosh) extracts in LPS-stimulated RAW 264.7 macrophages", JOURNAL OF PHARMACY AND PHARMACOLOGY, vol. 61, no. 8, August 2009 (2009-08), pages 1089-1096, XP009144216, ISSN: 0022-3573 * abstract * ----- -/-- | 2-8 | TECHNICAL FIELDS SEARCHED (IPC) |

The present search report has been drawn up for all claims

1

| Place of search | Date of completion of the search | Examiner |
|---|---|---|
| Munich | 8 February 2011 | Büttner, Ulf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 7121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | POWELL SHARLA L ET AL: "In Vitro Serotonergic Activity of Black Cohosh and Identification of N-omega-Methylserotonin as a Potential Active Constituent", JOURNAL OF AGRICULTURAL AND FOOD CHEMISTRY, vol. 56, no. 24, December 2008 (2008-12), pages 11718-11726, XP002620988, ISSN: 0021-8561 * table 1 * ----- | 1-8 | |
| A | HEDLUND PETER B: "The 5-HT7 receptor and disorders of the nervous system: an overview", PSYCHOPHARMACOLOGY, vol. 206, no. 3, October 2009 (2009-10), pages 345-354, XP002620989, ISSN: 0033-3158 * page 349 * * page 351 * ----- | 1-8 | TECHNICAL FIELDS SEARCHED (IPC) |
| A | MNIE-FILALI OUISSAME ET AL: "Therapeutic Potential of 5-HT7 Receptors in Mood Disorders", CURRENT DRUG TARGETS, vol. 10, no. 11, November 2009 (2009-11), pages 1109-1117, XP009144211, ISSN: 1389-4501 * page 1112 * ----- -/-- | 1-8 | |

The present search report has been drawn up for all claims

1

| Place of search | Date of completion of the search | Examiner |
|---|---|---|
| Munich | 8 February 2011 | Büttner, Ulf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 7121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | CICEK SERHAT S ET AL: "Bioactivity-guided isolation of GABA(A) receptor modulating constituents from the rhizomes of Actaea racemosa.", JOURNAL OF NATURAL PRODUCTS 27 DEC 2010 LNKD- PUBMED:21082802, vol. 73, no. 12, 27 December 2010 (2010-12-27), pages 2024-2028, XP002620991, ISSN: 1520-6025 * figure 1 * ----- | 1-8 | |

TECHNICAL FIELDS SEARCHED (IPC)

The present search report has been drawn up for all claims

1

| Place of search | Date of completion of the search | Examiner |
|---|---|---|
| Munich | 8 February 2011 | Büttner, Ulf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9031094 A


### Non-patent literature cited in the description


- **BRITTON, N et al.** An illustrated flora of the Northern United States, Canada and the British Possessions. Charles Scribner's sons, 1913, vol. II
- **HARDY, M.** *J. Am. Pharmaceut. Assoc.,* 2000, vol. 40, 234-242
- **FOSTER, S.** *Herbalgram,* 1999, 35-49
- **PALACIO, C et al.** *Drugs Aging,* 2009, vol. 26, 23-36
- **JARRY, H et al.** *Planta Med.,* 1985, 46-49
- **JARRY, H et al.** *Planta Mend.,* 1985, 316-319
- **DÜKER, EM et al.** *Planta Med.,* 1991, vol. 57, 420-424
- **WUTTKE, W et al.** *Maturitas,* 2003, vol. 44, 9-20
- **ZAVA, D.** *Proc. Soc. Exp. Biol, Med.,* 1998, vol. 217, 369-378
- **LIU, J et al.** *J. Agric. Food Chem.,* 2001, vol. 49, 2472-2479
- **ZIERAU, O et al.** *J. Steroid Biochem. Mol. Biol.,* 2002, vol. 80, 125-130
- **AMATO, P et al.** *Menopause,* 2002, vol. 9, 145-150
- **BODINET, C et al.** *Breast Cancer Res. Treat.,* 2002, vol. 76, 1-10
- **ONORATO, J et al.** *Anal. Chem.,* 2001, vol. 73, 4704-4710
- **BORELLI, F et al.** *Pharmacol. Res.,* 2008, vol. 58, 8-14
- **BUNDGAARD, H.** Design of Prodrugs. Elsevier, 1985, 7-921-24
- Remington's Pharmaceutical Sciences